# EUROPEAN PATENT SPECIFICATION

(11) **EP 0 674 631 B1**
(45) Date of publication and mention of the grant of the patent: **08.12.1999**
(21) Application number: 93922407.7
(22) Date of filing: 30.09.1993
(51) Int. Cl.: C07D 401/06, C07D 231/12, C07D 231/18, A61K 31/415, A61K 31/47

(54) **SUBSTITUTED PYRAZOLES AS CRF ANTAGONISTS**
SUBSTITUIERTE PYRAZOLE ALS CRF ANTAGONISTEN
PYRAZOLES SUBSTITUES UTILISES COMME ANTAGONISTES DU FACTEUR DE LIBERATION DE CORTICOTROPINE (CRF)

(30) Priority: 17.12.1992 US 992228
(43) Date of publication of application: 04.10.1995
(60) Divisional application: 99109027.5
(73) Proprietor: PFIZER INC., New York, N.Y. 10017 (US)
(72) Inventor: BRIGHT, Gene, M., Groton, CT 06340 (US); WELCH, Willard, M., Jr., Mystic, CT 06355 (US)
(74) Representative: Hayles, James Richard
(86) International application number: US9309170
(87) International publication number: WO9413661

(56) References cited:
- US-A- 5 063 245

## Description

This invention relates to substituted pyrazoles, pharmaceutical compositions containing them, and their use in the treatment of stress-related and other diseases. The compounds have corticotropin-releasing factor (CRF) antagonist activity.

CRF antagonists are mentioned in U.S. Patents 4,605,642 and 5,063,245 referring to peptides and pyrazolinones, respectively. The importance of CRF antagonists is set out in the literature, e.g. as discussed in U.S. Patent 5,063,245, which is incorporated herein by reference. A recent outline of the different activities possessed by CRF antagonists is found in M.J. Owens et al., Pharm. Rev., Vol. 43, pages 425 to 473 (1991), also incorporated herein by reference. Based on the research described in these two and other references, CRF antagonists are considered effective in the treatment of a wide range of diseases including stress-related illnesses, such as stress-induced depression, anxiety, and headache; abdominal bowel syndrome; inflammatory diseases; immune suppression; human immunedeficiency virus (HIV) infections; Alzheimer's disease; gastrointestinal diseases; anorexia nervosa; hemorrhagic stress; drug and alcohol withdrawal symptoms; drug addiction, and fertility problems.

The present invention relates to a compound of the formula 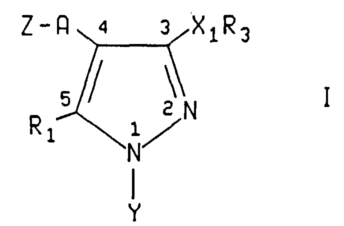 and the pharmaceutically acceptable acid addition salts thereof,
wherein
A is CH₂;
R₁ is hydrogen; linear or branched C₁-C₆ alkyl; C₃-C₆ alkyl containing one or two non-adjacent double bonds; hydroxy; O(C₁-C₆ alkyl); SH; S(C₁-C₆ alkyl); C₃-C₆ cycloalkyl; morpholinyl, piperidinyl or aryl which aryl may be substituted by one to three of fluoro, chloro, bromo, trifluoromethyl, hydroxy, O(C₁-C₆ alkyl), SH, S(C₁-C₆ alkyl), amino, NH(C₁-C₆ alkyl), N(C₁-C₆ alkyl)₂, or one of iodo, nitro or cyano, said aryl being selected from the group consisting of phenyl, thienyl, benzothienyl, pyridyl, quinolyl, pyrazinolyl, pyrimidyl, imidazolyl, benzimidazolyl, furanyl, benzofuranyl, thiazolyl, benzothiazolyl, isothiazolyl, benzoisothiazolyl, isoxazolyl, benzisoxazolyl, triazolyl, pyrazolyl, pyrrolyl, indolyl, azaindolyl, oxazolyl, benzoxazolyl, pyrrolidinyl, and thiazolidinyl;
R₃ is linear C₁-C₆ alkyl, branched C₃-C₈ alkyl, C₃-C₈ alkenyl wherein the double bond is not adjacent to X₁ when X₁ is a heteroatom, or C₃-C₇ cycloalkyl(CH₂)ₙ wherein n is 0 to 4, or (CH₂)_{q}Q₁R₁₉ wherein q is 0, 1 or 2, Q₁ is O, S, NH, N(C₁-C₆ alkyl), or a covalent bond when X₁ is not a covalent bond, and R₁₉ is hydrogen, linear C₁-C₆ alkyl, branched C₃-C₈, C₃-C₈ alkenyl, C₃-C₆ cycloalkyl or C₃-C₆ cycloalkyl (CH₂);
X₁ is a covalent bond, CH₂, O, S, or NR, wherein R is hydrogen or linear C₁-C₆ alkyl or branched C₃-C₈ alkyl;
Y is phenyl, thienyl, benzothienyl, pyridyl, quinolyl, pyrazinolyl, pyrimidyl, imidazolyl, benzimidazolyl, furanyl, benzofuranyl, thiazolyl, benzothiazolyl, isothiazolyl, benzisothiazolyl, isoxazolyl, benzisoxazolyl, triazolyl, pyrazolyl, pyrrolyl, indolyl, azaindolyl, oxazolyl, benzoxazolyl, pyrrolidinyl, thiazolidinyl, morpholinyl, or piperidinyl, each of which may be substituted by one to three of any one of fluoro, chloro, bromo, or methyl, or one of trifluoromethyl; with the proviso that Y is not unsubstituted phenyl; and
Z is 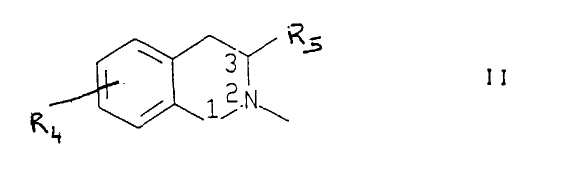
R₄ is hydrogen, C₁-C₆ alkyl, C₁-C₆ alkoxy, hydroxy, fluoro, chloro, bromo, iodo, or trifluoromethyl;
R₅ is hydrogen, linear C₁-C₆ alkyl, branched C₃-C₈ alkyl, C₃-C₈ alkenyl, or (CH₂)ₒ-X₂-(CH₂)ᵣ-Q₂-R₆;
X₂ and Q₂ are each independently O, S, NH, N(C₁-C₆ alkyl), or one of X₂ and Q₂ may be a covalent bond;
R₆ is hydrogen, linear C₁-C₆ alkyl, branched C₃-C₈ alkyl or C₃-C₈ alkenyl;
m is 0 or 1;
o is 1 or 2;
p is 1 or 2; and
r is 0, 1 or 2
or z is 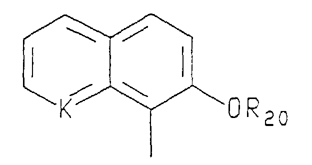
wherein K is C or N and R₂₀ is methyl, ethyl, isopropyl, cycloprapylmethylene, methoxyethylene, hydroxyethylene.

Preferred compounds of formula I are those wherein Z is 1,2,3,4-tetrahydroisoquinolin-2-yl substituted by R₅ which is (CH₂)ₒ-X₂-(CH₂)ᵣ-Q₂-R₆, or more preferably R₅ is (CH₂)ₖOH wherein k is 1 to 4, or CH₂OCHCH₂OR₆. Other preferred compounds are those wherein Z is 1,2,3,4-tetrahydroisoquinolin-2-yl, wherein R₅ is substituted at position 3, and the absolute configuration at the 3-position is S or R or R,S. Further preferred compounds are those wherein Z is of the formula 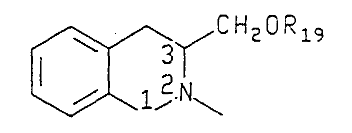 with the absolute configuration at position 3 determined by its derivation from (+)-3-hydroxymethyl-1,2,3,4-tetrahydroisoquinoline, wherein R₁₉ is methyl, ethyl, isopropyl, cyclopropylmethylene, or 2-hydroxyethyl, and, more preferably, wherein in addition XR₃ is ethyl or methylthio, Y is 2,6-dichloro-4-trifluoromethylphenyl, 2,4,6-trichlorophenyl, 2,4,6-trimethylphenyl, 2,6-dimethyl-4-bromophenyl, or 2,6-dibromo-4-fluorophenyl, and R₁ is methyl or ethyl.

More specific compounds of formula I include those wherein Z is 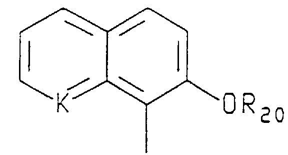 wherein K is C or N and R₂₀ is methyl, ethyl, isopropyl, cyclopropylmethylene, methoxyethylene, hydroxyethylene, and, more specifically, in addition X₁R₃ is ethyl or methylthio, Y is 2,6-dichloro-4-trifluoromethylphenyl, 2,4,6-trichlorophenyl or 2,6-dibromo-4-fluorophenyl, and R₁ and R₂ are each methyl or ethyl.

Other more specific compounds are those of formula I wherein Z is as defined above and R₆ is CH₂OCH₃ or CH₂OCH₂CH₂OH.

More specific compounds of formula I of the invention include those wherein Y is phenyl substituted by three substituents one each at positions 2, 4 and 6, e.g. 2,4,6-trichlorophenyl, 2,6-dimethyl-4-bromophenyl, 2,6-dichloro-4-trifluoromethylphenyl, 2,6-dichloro-4-fluorophenyl or 2,4,6-trimethylphenyl. Other more specific compounds of formula I include those wherein X₁R₃ is ethyl or methylthio, those wherein R₁ is (C₁-C₆) alkyl.

Specific, preferred compounds of formula I include-3-methoxymethyl-2-[5-methyl-3-methylsulfanyl-1-(2,4,6-trichlorophenyl)-1H-pyrazol-4-ylmethyl]-1,2,3,4-tetrahydroisoquinoline; (3R)-3-methoxymethyl-2-[5-methyl-3-methylsulfanyl-1-(2,4,6-trichlorophenyl)-1H-pyrazol-4-ylmethyl]-1,2,3,4-tetrahydroisoquinoline; 3-methoxymethyl-2-[5-methyl-3-methylsulfanyl-1-(2,6-dichloro-4-trifluoromethylphenyl)-1H-pyrazol-4-ylmethyl]-1,2,3,4-tetrahydroisoquinoiine; {2-[5-methyl-3-methylsulfanyl-1-(2,4,6-trichlorophenyl)-1H-pyrazol-4-ylmethyl]-1,2,3,4-tetrahydroisoquinolin-3-yl}methanol; {2-[5-methyl-3-methylsulfanyl-1-(2,6-dichloro-4-trifluoromethylphenyl)-1H-pyrazol-4-ylmethyl]-1,2,3,4-tetrahydroisoquinolin-3-yl}methanol; 2-{1-(2,6-dichloro-4-trifluoromethylphenyl)-3,5-diethyl-1H-pyrazol-4-ylmethyl]-naphthalene-2-yloxy}-ethanol; 2-{8[1-(2,6-dichloro-4-trifluoromethylphenyl)-3,5-diethyl-1H-pyrazol-4-ylmethyl]-quinolin-7-yloxy}-ethanol; 2-[3,5-diethyl-1-(2,4,6-trichlorophenyl)-1H-pyrazol-4-ylmethyl]-3-methoxymethyl-1,2,3,4-tetrahydroisoquinoline; or 1-(2,6-dichloro-4-trifluoromethylphenyl)-3,5-diethyl-4-(2-methoxynaphthalen-1-ylmethyl)-1H-pyrazole; 2-{2-[1-(2,6-dichloro-4-trifluoromethylphenyl)-3,5-diethyl-1H-pyrazol-4-ylmethyl]-1,2,3,4-tetrahydro-isoquinolin-3-ylmethoxy}-ethanol; 2-{1-[3,5-diethyl-1-(2,4,6-trimethylphenyl)-1H-pyrazol-4-ylmethyl]naphthalen-2-yloxy}-ethanol; 2-[1-(4-bromo-2,6-dimethylphenyl)-3,5-diethyl-1H-pyrazol-4-ylmethyl]-3-methoxymethyl- 1,2,3,4-tetrahydroisoquinoline; 2-[1-(4-bromo-2,6-dimethylphenyl)-3,5-diethyl-1H-pyrazol-4-yimethyl]-3-ethoxymethyl-1,2,3,4-tetrahydroisoquinoline; and 2-{2-[3,5-diethyl-1-(2,4,6-trimethylphenyl)-1H-pyrazol-4-ylmethyl]-1,2,3,4-tetrahydro-isoquinolin-3-ylmethoxy}-ethanol.

Specific, most preferred compounds of formula I include 2-[1-(2,6-dichloro-4-trifluoromethylphenyl)-3,5-diethyl-1H-pyrazol-4-ylmethyl]-3-methoxymethyl-1,2,3,4-tehrahydroisoquinoline, 2-[3,5-diethyl-1-(2,4,5-trímethylphenyl)-1H-pyrazol-4-ylmethyl]-3-ethoxymethyl-1,2,3,4-tetrahydroisoquinoline, 2-[1-(2,6-dichloro-4-trifluoromethylphenyl)-3,5-diethyl-1H-pyrazol-4-ylmethyl]-3-methoxymethyl-1,2,3,4-tetrahydrosioquinoline, and 2-[3,5-diethyl-1-(2,4,6-trimethylphenyl)-1H-pyrazol-4-ylmethyl]-3-ethoxymethyl-1,2,3,4-tetrahydroisoquinoline.

The invention also relates to a pharmaceutical composition for the treatment of (a) illnesses induced or facilitated by corticotropin releasing factor or (b) stress and anxiety related disorders, including stress-induced depression and headache, abdominal bowel syndrome, immune suppression, HIV infections, Alzheimer's disease, gastrointestinal disease, anorexia nervosa, hemorrhagic stress, drug and alcohol withdrawal symptoms, drug addiction, and fertility problems, which comprises a compound of the formula I or IA as defined above in an amount effective in the treatment of said illnesses or disorders, and a pharmaceutically acceptable carrier. Preferred compositions of the invention are those containing preferred compounds of formula I as described above.

The invention further relates to use in a method for the treatment of illnesses induced or facilitated by corticotropin releasing factor by administering to a subject in need of such treatment a compound of formula I as defined above in an amount effective in such treatment, and a method for the treatment of stress and anxiety related disorders, including stress-induced depression and headache, abdominal bowel syndrome, inflammatory disorders, immune suppression, HIV infections, Alzheimer's disease, gastrointestinal diseases, anorexia nervosa, hemorrhagic stress, drug and alcohol withdrawal symtoms, drug addiction, and fertility problems, particularly depression, by administering to a subject in need of such treatment a compound of formula I as defined above in an amount effective in such treatment. Preferred methods of the invention are those administering a preferred compound of the formula I as described above.

The invention also relates to an intermediate compound of the formula 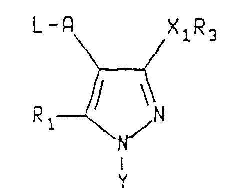 wherein
A is CH₂, R₃ is linear C₁-C₆ alkyl, branched C₃-C₈ alkyl, C₃-C₈ alkenyl wherein the double bond is not adjacent to the N or X₁ when X₁ is oxygen or sulfur, C₃-C₇ cycloalkyl (CH₂)ₙ wherein n is 0, 1, 2, 3 or 4; or (CH₂)_{q}Q₁R₆ wherein q is 0, 1 or 2, Q₁ is O, S, NH, N(C₁-C₆ alkyl) or a covalent bond, and R₆ is hydrogen, linear C₁-C₆ alkyl, branched C₃-C₈ alkyl, C₃-C₈ alkenyl, C₃-C₆ cycloalkyl, or C₃-C₆ cycloalkyl (CH₂)ₙ wherein n is 0 to 4, with the proviso that when q is 1, then X₁ and Q₁ can not both be a heteroatom;
X₁ is a covalent bond, CH₂NR, wherein R is hydrogen or linear C₁-C₆ alkyl, O, or S;
Y is phenyl, thienyl, benzothienyl, pyridyl, quinolyl, pyrazinolyl, pyrimidyl, imidazolyl, benzimidazolyl, furanyl, benzofuranyl, thiazolyl, benzothiazolyl, isothiazolyl, benzisothiazolyl, isoxazolyl, benzisoxazolyl, triazolyl, pyrazolyl, pyrrollyl, indolyl, azaindolyl, oxazolyl, benzoxazolyl, pyrrolidinyl, thiazolidnyl, morpholinyl, or piperidinyl, each of which may be substituted by one to three of any one of fluoro, chloro, bromo, or methyl, or one of trifluoromethyl, provided that Y is not unsubstituted phenyl, and
L is chloro, bromo, iodo, hydroxy, O(C=O)(C₁-C₆ alkyl), OSO₂(C₁-C₆ alkyl), OSO₂aryl wherein said aryl is phenyl which may be substituted by one to three of fluoro, chloro, bromo, hydroxy, O(C₁-C₆ alkyl), SH, S(C₁-C₆ alkyl), amino, NH(C₁-C₆ alkyl), N(C₁-C₆ alkyl)₂, or one of iodo, nitro or cyano.

Whenever reference herein is made to the groups (CH₂)_{q}Q₁R₁₉ and (CH₂)_{O}-X₂-(CH₂)_{f}Q₂R₆, then X₁ and Q₁, and X₂ and Q₂, respectively, are not both a heteroatom when q or r, respectively, is 1.

Whenever R₁ or Y is a heterocyclic group, the attachment of the group is through a carbon atom.

The compounds of formula I may be prepared by reaction of a compound of the formula 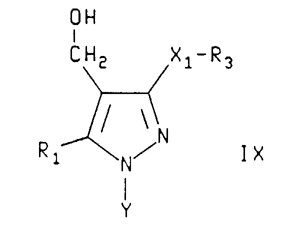 wherein R₁ and Y are as defined above with reference to formula I , with a compound of the formula ZH wherein Z is as defined above.

This reaction generally proceeds at temperatures ranging from about 0° to 85°C, usually at room temperature. The reaction is conveniently carried out in a solvent which is inert under the reaction conditions, e.g. acetonitrile. The compound of formula IX is first reacted with an activated sulfonic acid such as methylsulfonyl chloride in the presence of an acid neutralizing agent such as triethylamine in an inert solvent such as methylene chloride at about -10° to about 50°C, before reaction with ZH.

The compounds of formula IX may be prepared by reacting a compound of the formula 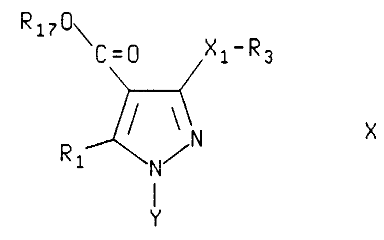 wherein R₁, X₁ and Y are as defined with reference to formula I and R₁₇ is C₁-C₆ alkyl, with a reducing agent such as diisobutylaluminum hydride at temperatures of about -10° to about 80°C, in a reaction-insert solvent such as tetrahydrofuran or ether.

The compounds of formula X may be prepared by reaction of a compound of the formula 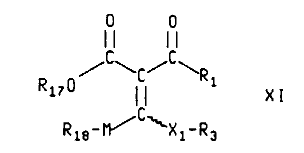 with a compound of the formula Y-NHNH₂, wherein X₁, R₁, R₃ and Y are as defined with reference to formula I, M is O or S, R₁₇ is as defined above with reference to formula X, and R₁₈ is C₁-C₆ alkyl. The reaction is usually carried out in a solvent, such as a C₁-C₈ alcohol, at least 50 to 150°C, conveniently the reflux temperature of the reaction mixture. The wavy line 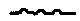 in formula XI indicates that either isomer of this compound is included, in accordance with accepted conventions for indicating stereoisomers.

The compounds of formula Xl above may be prepared by reacting an appropriate beta-ketoester with a base such as sodium hydride in the presence of carbon disulfide in an appropriate solvent or mixture of solvents such as dimethylsulfoxide or dimethylformamide at a temperature of about -10° to about 40°C followed by quenching of the resulting dianion with an appropriate alkylating agent such as methyl iodide resulting in a 3,3-bismethylthioacrylate derivative XI wherein R₁₈ is R₃ is CH₃ and M is X₁ is S. Reaction of compounds of the formula XI wherein M is X₁ is S and R₃ is R₁₈ is C₁-C₆ alkyl with alcohols R₃OH in the presence of base then results in the preparation of the corresponding compounds XI wherein R₁₈ is R₃ and M is X₁ is O.

Reaction of an appropriate beta-ketoester with an ortho ester of one of the following formulas:

(C₁-C₆ alkyl)-(CH₂)ₙ-C[O-(C₁C₆ alkyl)]₃;

(C₂-C₈ alkenyl)-(CH₂)ₙ-C[O-(C₁C₆ alkyl)]₃;

or

R₁₉Q₁(CH₂)_{q}-X₁-(CH₂)ₙ-C[O-(C₁-C₆alkyl)]₃,

wherein n, R₁₉, Q₁, q, and X₁ are as defined with reference to formula I, in an appropriate solvent such as ethyl acetate at temperatures of about 0° to about 100°C results in compounds of the formula XI wherein R₁₈ is C₁-C₆ alkyl, M is O, X₁ is CH₂ or a covalent bond, and R₃ is, respectively, (C₁-C₆ alkyl)-(CH₂)ₙ; (C₂-C₈) alkenyl)-(CH₂)ₙ; and R₁₉Q₁(CH₂)_{q}-X₁-(CH₂)ₙ, wherein n, q, R₁₉, Q₁ and X₁ are as defined above.

Reaction of the compounds of the formula Xl wherein M is X₁ is S and R₃ is R₁₈ is C₁-C₆ alkyl with amines such as RNH₂ or RR₃NH in an appropriate solvent such as ethanol at temperatures of about 0° to about 100°C results in compounds of the formula XI in which either or both of R₁₈-M and X₁-R₃ are each RNH or NRR₃, wherein R is as defined with reference to formula I and R₃ is linear alkyl, branched C₃-C₈ alkyl, or C₃-C₈ alkenyl wherein the double bond is not adjacent to the nitrogen.

The compounds of formula I wherein Z is as defined above in paragraphs (a), (h) or (i) wherein R₅ or R₁₄ is X₂(CH₂)ᵣQ₂R₆, wherein Q₂ is oxygen, and X₂, r, and R₆ are as previously defined except that R₆ is not hydrogen, may be prepared by alkylation of the corresponding compound wherein R₆ or R₁₄ are (CH₂)ₒ-X₂-(CH₂)₂-Q₂-R₆ and -X₂-(CH₂)ᵣQ₂R₆, respectively, wherein R₆ is hydrogen and Q₂ is oxygen. In these cases wherein R₅ have a terminal hydroxy group, the hydroxy is first reacted with a strong base such as an alkali metal hydride, e.g. lithium, sodium or potassium hydride, in a solvent such as dimethylformamide at about 50° to 100°C.

The resulting alkali metal alkoxide is then reacted with an alkyl or aryl sulfonyl ester of the formula HO(CH₂)ᵣQ₂R₆ wherein R₆ is as defined in paragraph (a) except hydrogen. This reaction is carried out in the presence of a solvent such as methylene chloride or toluene at about 50° to 100°C. The above sulfonyl esters may be prepared by the same method as described above for the activation of the compound of formula IX.

The above alkali metal hydride may be replaced by other strong bases including organometallic bases such as n-butyl lithium or amine anion bases such as lithium diisopropylamide. In such case, the metal alkoxide formation reaction may be carried out in tetrahydofuran at temperatures of about -5° to about 65°C.

The same alkylation may be used to prepare compounds of the formula I wherein X₁ is oxygen and R₃ is (CH₂)_{q}Q₁R₁₉ wherein q, Q₁ and R₁₉ are as defined above with reference to formula I except that R₁₉ is not hydroxy, from the corresponding compounds wherein X₁R₃ is hydroxy.

The compounds of the formula IX wherein R₃ is (CH₂)_{q}Q₁R₆ Wherein q is as defined with reference to formula I, Q₁ is O and R₆ is methyl, react with ZH, as defined above, to form compounds of the formula 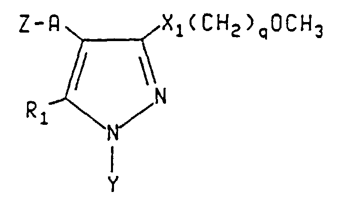 These compounds may be reacted with a demethylating agent to form the corresponding compound wherein R₆ is hydrogen. A suitable demethylating agent is boron tribromide in combination with sodium iodide and 15-crown-5, as described in the prior art.

When the compounds of the invention contain a chiral center, it is understood that the invention includes the racemic mixture and the individual enantiomers of such compounds. For instance, the compounds of the invention wherein Z is 1,2,3,4tetrahydroisoquinolinyl have a chiral center when Z is substituted at position 3 by R₅, wherein R₅ is as defined with reference to formula I except hydrogen, as follows: 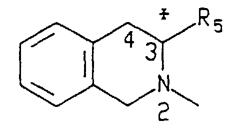 wherein the chiral center is indicated by an asterisk.

Preferred compounds of the invention of formula I include those derived from the dextrorotatory (+) enantiomer of the intermediate compound ZH of the formula 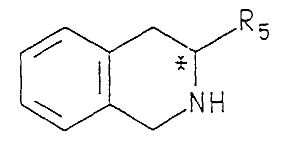 wherein R₅ is hydroxymethyl or (C₁-C₆ alkoxy) methyl.

The acid addition salts are prepared in a conventional manner by treating a solution or suspension of the free base of formula I with one chemical equivalent of a pharmaceutically acceptable acid. Conventional concentration or crystallization techniques are employed in isolating the salts. Illustrative of suitable acids are acetic, lactic, succinic, maleic, tartaric, citric, gluconic, ascorbic, benzoic, cinnamic, fumaric, sulfuric, phosphoric, hydrochloric, hydrobromic, hydroiodic, sulfamic, sulfonic acids such as methanesulfonic, benzene sulfonic, p-toluenesulfonic, and related acids.

The novel compounds of the invention of formula I may be administered alone or in combination with pharmaceutically acceptable carriers, in either single or multiple doses. Suitable pharmaceutical carriers include inert solid diluents or fillers, sterile aqueous solution and various organic solvents. The pharmaceutical compositions formed by combining the novel compounds of formula I and the pharmaceutically acceptable carriers are then readily administered in a variety of dosage forms such as tablets, powders, lozenges, syrups and injectable solutions. These pharmaceutical compositions can, if desired, contain additional ingredients such as flavorings, binders and excipients. Thus, for purposes of oral administration, tablets containing various excipients such as sodium citrate, calcium carbonate and calcium phosphate may be employed along with various disintegrants such as starch, alginic acid and certain complex silicates, together with binding agents such as polyvinylpyrrolidone, sucrose, gelatin and acacia. Additionally, lubricating agents such as magnesium stearate, sodium lauryl sulfate and talc are often useful for tabletting purposes. Solid compositions of a similar type may also be employed as fillers in soft and hard filled gelatin capsules. Preferred materials for this include lactose or milk sugar and high molecular weight polyethylene glycols. When aqueous suspensions or elixirs are desired for oral administration, the essential active ingredient therein may be combined with various sweetening or flavoring agents, coloring matter or dyes and, if desired, emulsifying or suspending agents, together with diluents such as water, ethanol, propylene glycol, glycerin and combinations thereof.

For parenteral administration, solutions of the novel compound of formula I in sesame or peanut oil, aqueous propylene glycol, or in sterile aqueous solution may be employed. Such aqueous solutions should be suitably buffered if necessary and the liquid diluent first rendered isotonic with sufficient saline or glucose. These particular aqueous solutions are especially suitable for intravenous, intramuscular, subcutaneous and intraperitoneal administration. The sterile aqueous media employed are all readily available by standard techniques known to those skilled in the art.

Additionally, it is possible to administer the compounds of the present invention topically when treating inflammatory conditions of the skin and this may be done by way of creams, jellies, gels, pastes, and ointments, in accordance with standard pharmaceutical practice.

The effective dosage for the compound of formula I depends on the intended route of administration and other factors such as age and weight of the patient, as generally known to a physician. The dosage also depends on the illness to be treated. The daily dosage will generally range from about 0.1 to 50 mg/kg of the body weight of the patient to be treated. For the treatment of inflammatory diseases about 0.1 to about 100 mg/kg will be needed in general, for gastrointestinal diseases about 0.1 to about 50 mg/kg, as well as for anorexia nervosa, hemorrhagic stress, treatment of drug and alcohol withdrawal symptoms and treatment of fertility problems. The daily dosage may be given in a single dose or up to three divided doses.

The methods for testing the compounds of formula I for their CRF antagonist activity are as described in Endocrinology, 116, 1653-1659 (1985) and Peptides 10, 179-188 (1989) which determine the binding activity of a test compound to a CRF receptor. The binding activity for the compounds of formula I generally ranges from about 0.2 nanomolar to about 10 micromolar.

The following Examples illustrate the invention. The designation Et means ethyl.

### EXAMPLE I

### A. Ethyl 3,3-bismethylthio-2-acetylacrylate.

A solution of 6.50 g (50.0 mmol) of ethyl acetoacetate and 4.18 g (3.30 mL, 55.0 mmol) of carbon disulfide in 60 mL of dry dimethylsulfoxide in a flame-dried 300 mL flask was treated portionwise at 16 - 18°C with 2.64 g (110 mmol) of oil-free sodium hydride. An additional 100 mL of dimethylsulfoxide was eventually added to facilitate stirring. After the addition was complete, the deep red solution was stirred for 75 minutes and then was quenched with 15.62 g (6.85 mL, 110 mmol) of methyl iodide. The reaction mixture was stirred overnight at room temperature. The solution was poured into water and extracted with ether. The extracts were washed with water, dried and evaporated to give a red oil which was used for subsequent reactions without further purification. ¹H-NMR (CDCl₃) δ 1.24 (3H, t, J=7), 2.28 (3H, s), 2.37 (6H, s), 4.21 (2H, q, J=7).

### B. 4-Ethoxycarbonyl-5-methyl-3-methylthio-1-(2,4,6-trichlorophenyl)pyrazole.

A mixture of 1.22 g (5.23 mmol) of ethyl 3,3-bismethylthio-2-acetylacrylate and 1.11 g (5.23 mmol) of 2,4,6-trichlorophenylhydrazine in 12 mL of ethanol was heated at reflux for 2 hours. The cooled reaction mixture was then poured into cold water and the product was extracted into ether. The ethereal extracts were dried and evaporated and the residues were chromatographed on silica gel using 6:1 hexane/ethyl acetate as eluent to give 1.12 g (56%) of the desired product as a crystalline solid, m.p. 95-98°C. ¹H-NMR (CDCl₃) δ 1.38 (3H, t, J=7), 2.30 (3H, s), 2.49 (3H, s.), 4.31 (2H, q, J=7), 7.47 m(2H, s).

### C. 2-(5-Methyl-3-methylthio-1-(2,4,6-trichlorophenyl)pyrazol-4-yl)methyl-1,2,3,4-tetrahydroisoquinoline.

A solution of 0.340 g (0.89 mmol) of 4-ethoxycarbonyl-5-methyl-3-methylthio-1-(2,3,6-trichlorophenyl)pyrazole in 10 mL of tetrahydrofuran was cooled to 0°C in an ice bath under dry nitrogen and then 2.37 mL of a 1.5 M solution of diisobutylaluminum hydride in toluene (3.56 mmol) was added. The reaction mixture was allowed to warm to room temperature and stir for 2 hours. Then water was added cautiously and the product was extracted into ether which was dried and evaporated to give the product which was used for the subsequent reaction without further purification. ¹H-NMR (CDCl₃) δ 2.07 (3H, s), 2.53 (3H, s), 4.56 (2H, d, J=7), 7.45 (2H, s).

The above product was dissolved in 10 mL of methylene chloride and 0.62 mL (0.45 g, 4.45 mmol) of triethylamine at 0 - 5°C and treated with 0.21 mL (0.31 g, 2.67 mmol) of methanesulfonyl chloride. After 1 hour at room temperature, the reaction mixture was poured into water and was extracted with ethyl acetate. The solution of product was dried with brine and magnesium sulfate and the solvent was evaporated to give the intermediate mesylate which was used in the subsequent step without further purification.

The product of the above reaction (0.98 mmol) was dissolved in 10 mL of acetonitrile and treated with 0.45 mL (0.475 g, 3.57 mmol) of 1,2,3,4-tetrahydroisoquinoline. The solution darkened and then lightened over a period of a few minutes and was then stirred overnight at room temperature. Solids which had formed were filtered off and discarded and the filtrate was concentrated and chromatographed on silica gel using 4:1 hexane/ethyl acetate as eluent to give the product free base. This material was dissolved in ether and treated with a solution of hydrogen chloride (gas) in ether to give the product hydrochloride, m.p. 205-207°C (53% over the three reactions). Anal. Calcd for C₂₁H₂₀N₃SCl₃: C, 51.55; H, 4.33; N, 8.59. Found, C, 51.01; H, 4.69; N, 8.40.

### EXAMPLE 2

The following compounds were prepared by the process of Example 1.

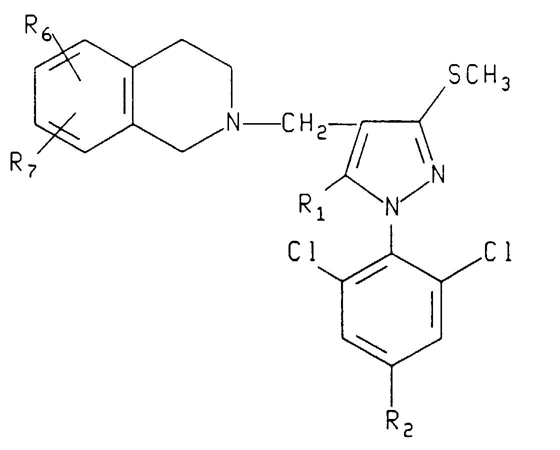

| | | | | |
|---|---|---|---|---|
| R₁ | R₂ | R₆ | R₇ | Physical data (m.p. in °C) |
| CH₃ | Cl | H | H | m.p. 205-207 |
| CH(CH₃)₂ | Cl | H | H | m.p. 209-210 |
| CH(CH₃)₂ | Cl | OCH₃ | OCH₃ | m.p. 140-142 |
| phenyl | CF₃ | H | H | ¹H-NMR(ODCl₃) δ 2.59 (s, 3H), 2.74 (2H, t, J=7), 2.89 (2H, t, J=7), 3.54 (2H, s), 3.64 (2H, s), 6.98-7.01 (1H, m), 7.07-7.15 (3H, m), 7.25-7.32 (3H, m), 7.37-7.42 (2H, m), 7.60 (2H, m). |

### EXAMPLE 3

### A. 4-Methoxycarbonyl-3,5-heptanedione.

A solution of 6.5 g (50 mmol) of methyl propionyl acetate in 100 mL of ether was treated with 1.19 g (50 mmol) of sodium hydride and the mixture was stirred for 2 hours. The mixture was then cooled to 5 °C and 6.93 g (6.51 mL. 75 mmol) of propionyl chloride was added dropwise over 5 minutes. The reaction mixture was stirred overnight at room temperature and then poured into cold water. This mixture was acidified with sulfuric acid and the product was extracted into ether, washed with water and dried. Evaporation gave the desired product, sufficiently pure for use in the following reaction, in 88% yield. ¹H-NMR (CDCl₃) δ 1.08 (6H, t, J=7), 2.58 (4H, q. J=7), 3.66 (1H, s), 3.74 (3H, s).

### B. Methyl 1-(2,6-dichloro-4-trifluoromethylphenyl)-3,5-diethylpyrazole-4-carboxylate.

A solution of 7.5 g (40 mmol) of the compound of step A and 11.85 g (48 mmol) of 2,6-dichloro4-trifluoromethylphenyl hydrazine in 50 mL of ethanol was heated at reflux for 8 hours. The ethanol was removed by evaporation and the residues were partitioned between ethyl acetate and dilute hydrogen chloride. The organic extracts were dried and evaporated to give the desired product in 43% yield as a maroon oil. ¹H-NMR (CDCl₃) δ 1.08 (3H, t, J=7), 1.24 (3H, t, J=7), 2.22 (2H, q, J=7), 2.94 (2H, q, J=7), 3.86 (3H, s), 7.46 (2H, s).

### C. [1-(2,6-Dichloro-4-trifluoromethylphenyl)-3,5-diethyl-1H-pyrazol-4-yl]methanol.

A solution of 8 g (20 mmol) of the compound of step B in 50 mL of tetrahydrofuran (THF) was treated at °C with 44.1 mL of 1.5 M diisobutylaluminum hydride in toluene solution over a period of 5 minutes. The reaction was stirred for 2 hours at °C and was then cautiously quenched with water. The product was extracted into ethyl acetate and dried and evaporated to give the title compound in 46% yield. ¹H-NMR (CDCl₃) δ 1.04 (3H, t, J=7), 1.26 (3H, t, J=7), 2.44 (2H, q, J=7), 2.70 (2H, q, J=7), 4.54 (2H, s), 7.66 (2H, s).

### D. 1-[3,5-Diethyl-1-(2,6-dichloro-4-trifluoromethylphenyl)-1H-pyrazol-4-ylmethyl]naphthalen-2-ol.

A solution of 303 mg (2.1 mmol) of 2-naphthol in 5 mL of dry ether was treated with 50 mg (2.1 mmol) of sodium hydride and the mixture was stirred for 15 minutes. A solution of 368 mg (1.0 mmol) of the compound of step C in 5 mL of dry ether and 126 mg (0.174 mL, 1.22 mmol) of triethylamine was cooled to 0°C and treated with 114 mg (0.077 mL, 1.0 mmol) of methanesulfonyl chloride. Triethylamine hydrochloride was removed by filtration and the filtrate was added to the above suspension of sodium 2-naphthoxide and the reaction mixture was stirred at room temperature for 12 hours. The reaction mixture was then partitioned between water and ether and the organic extracts were dried and evaporated to give the desired product in 29% yield. ¹H-NMR (CDCl₃) δ 1.00 (3H, t, J=7), 1.20 (3H, t, J=7), 2.44 (2H, q, J=7), 2.72 (2H, q, J=7), 4.58 (2H, s), 6.96 - 7.84 (8H, m).

### E. 3,5-Diethyl-4-(2-methoxynaphthalen-1-ylmethyl)-1-(2,6-dichloro-4-trifluoromethylphenyl)-1H-pyrazole.

A solution of 100 mg (0, 20 mmol) of the compound at step D in 5 mL of dry THF was treated with 5 mg (0.20 mmol) of sodium hydride and stirred for 15 minutes. Then 85 mg (0.037 mL, 0.60 mmol) of methyl iodide was added and the mixture was stirred overnight at room temperature. The reaction mixture was quenched with water and the product was extracted into ethyl acetate, dried and evaporated. Flash column chromatography gave the desired product as a white solid, m.p. 96 - 98°C. ¹H-NMR (CDCl₃) δ 0.6 (3H, t, J=7), 1.04 (3H, t, J=7), 206 (2H, q, J=7), 251 (2H, q, J=7), 3.90 (3H, s), 4.14 (2H, s), 7.18 - 7.34 (3H, m), 7.58 (2H, s, 7.70 - 7.84 (3H, m).

### EXAMPLE 4

### 8-[1-(2,6-Dichloro-4-trifluoromethylphenyl)-3,5-diethyl-1H-pyrazol-4-ylmethyl]quinolin-7-ol

By the general method of Example 3D, substituting 7-hydroxisoquinoline for 2-naphthol, the title compound was prepared [45 mg of an oil, isolated after flash chromatography (silica gel, 40 micron mesh; elution with ethylacetate/hexane=1:4 in volume), from reaction utilizing 264 mg (0.75 mmol) of the compound of Example 3C as starting material. ¹H-NMR(CDCl₃): 0.83 (3H, t), 1.09 (3H, t), 2.37 (2H, q), 2.50 (2H, q), 4.64 (2H, s), 7.14 (1H, d), 7.30 (1H, dd), 7.64 (1H, d), 770 (2H, s).

### EXAMPLE 5

### A. 2-{1-[1-(2,6-Dichloro-4-trifluoromethylphenyl)-3,5-diethyl-1H-pyrazol-4-ylmethyl]-napthalen-2-yloxy}-ethanol tert-butyl-dimethylsilylether

To a tetrahydrofuran (1.0 ml) solution of the compound of Example 3D (150 mg, 0.30 mmol), sodium hydride (37 mg of 60% sodium hydride mineral oil dispersion; 22.2 mg, 0.93 mmol of sodium hydride) was added portionwise over several minutes; 1-iodo-2-(tert-butyldimethylsilyloxy)ethane (858 mg, 0.30 mmol) was added, and the reaction was stirred and heated at 45°C for 48 hours. An additional (858 mg, 0.30 mmol) portion of 1-iodo-2-(tert-butyldimethylsilyloxy)ethane was added; and the reaction was then heated at 45°C for an additional 18 hours. The solvent was removed in vacuo, and the residue was extracted into ethyl acetate/water (100 ml of each). The separated aqueous layer was extracted twice with 30 ml portions of ethyl acetate. The combined organic extracts were dried (anhydrous sodium sulfate) and concentrated in vacuo to an oil (1.95 g). Flash chromatography of the entire sample (silica gel, 40 micron mesh; elution with ethyl acetate/hexane=5:95 in volume) afforded the title compound (40 mg) as an oil. ¹HNMR(CDCl₃): 0.10(6H, s), 0.60 (3H, t), 0.90 (9H, s), 1.10 (3H, t), 2.10 (2H, q), 2.56 (2H, q), 4.00 (2H, q), 4.20 (2H, q), 4.32 (2H, s), 7.25-7.38 (3H, m), 7.65 (2H, s), 7.73-7.87 (3H, m).

### B. 2-{1-[1-(2,6-Dichloro-4-trifluoromethylphenyl)-3,5-diethyl-1H-pyrazol-4-ylmethyl]-napthalen-2-yloxy}-ethanol

A tetrahydrofuran (0.40 ml) solution of the compound of step A, (40 mg, 0.06 mmol) and tetrabutylammonium fluoride (123 µl of a 1.00 M tetrahydrofuran (THF) solution, 0.123 mmol) was stirred at ambient temperature for 3 hours. The solvent was removed in vacuo, and the residue was extracted into ethyl acetate/water (60 ml of each). The separated organic phase was extracted twice with equal volume portions of water, dried over anhydrous sodium sulfate, and concentrated in vacuo to an oil (49 mg). Flash chromatography of the entire sample (silica gel, 40 micron mesh; elution with ethylacetate/hexane=3:7 in volume) afforded the title compound (24 mg) as an amorphous solid. ¹H-NMR(CDCl₃): 0.58(3H,t), 1.15 (3H, t), 1.99 (1H, broad), 2.07 (2H, q), 2.58 (2H, q), 3.99 (2H, m), 4.23 (2H, t), 4.32 (2H, s), 7.2-7.45 (3H, overlapping multiplets), 7.66 (2H, s), 7.80 (2H, dd), 7.91 (1H, d).

### EXAMPLE 6

### A. {2-[1-(2,6-Dichloro-4-trifluoromethylphenyl)-3,5-diethyl-1H-pyrazol-4-ylmethyl]-1,2,3,4-tetrahydroisoquinolin-3-yl}methanol

A solution of 368 mg (1.0 mmol) of [1-(2,6-dichloro-4-trifluoromethylphenyl)-3,5-diethyl-1H-pyrazol-4-yl]methanol in 10 mL of methylene chloride and 0.2 mL (2.5 mmol) of triethylamine was cooled to 0 - 5 °C. To this was added 0.92 mL (1.2 mmol) of methanesulfonyl chloride and the reaction mixture was stirred at 0 - 5 °C for 15 minutes. Then 1 mL of acetonitrile and 1 mL of dimethylformamide was added and the reaction mixture was heated at reflux overnight. The cooled reaction mixture was taken up with water and with ethyl acetate and the organic extracts were dried and evaporated to an orange oil which was purified by flash chromatography to give the desired product in 45% yield. ¹H-NMR (CDCl₃) δ 0.86 (3H, t, J=7), 1.21 (3H, t, J=7), 2.28 (2H, q, J=7), 2.60 (2H, q, J=7), 2.92-3.04 (1H, m), 3.20-3.32 (1H, m), 3.50-3.90 7H, m), 6.90-7.24 (4H, m), 7.68 (2H, s).

### B. 2-[1-(2,6-Dichloro-4-trifluoromethylphenyl)-3,5-diethyl-1H-pyrazol-4-ylmethyl]-3-methoxymethyl-1,2,3,4-tetrahydroisoquinoline

A solution of 200 mg (0.39 mmol) of the compound of step A in 5 mL of THF was treated with 10 mg (0.42 mmol) of sodium hydride and stirred for 30 minutes at room temperature. Then 0.1 mL (1.6 mmol) of methyl iodide was added and the reaction mixture was stirred at room temperature for 24 hours. The reaction was quenched with water and the product was extracted into ethyl acetate which was dried and evaporated. The crude product was flash chromatographed on silica gel to give the desired product in 26% yield as a colorless oil. ¹H-NMR (CDCl₃) δ 0.90 (3H, t, J=7), 1.20 (3H, t, J=7), 2.39 (2H, q, J=7), 2.65 (2H, q, J=7), 2.88-2.96 (1H, m), 3.16-3.20 (1H, m), 3.32 (3H, s), 3.55-3.78 7H, m), 6.90-7.24 (4H, m), 7.65 (2H, s).

### EXAMPLE 7

The following compounds were prepared according to the process of Example 6.

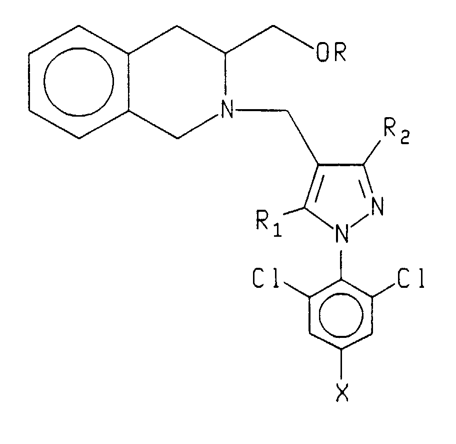

| | R | R₁ | R₂ | X | ¹H-NMR |
|---|---|---|---|---|---|
| Racemate | H | CH₃ | SCH₃ | Cl | (CDCl₃) δ 1.84 (3H, s), 2.48 (3H, s), 2.88 (2H, d of d, J=7,7), 3.22 (1H, m), 3.40-3.66 (5H, m), 3.79 (1H, d, J=7), 6.88-7.14 (4H, m), 7.40 (2H, s). |
| Racemate | CH₃ | CH₃ | SCH₃ | Cl | (CDCl₃) δ 1.96 (3H, s), 2.46 (3H, s), 2.80 (1H, ab quartet, J=7.2), 2.82 (1H, ab quartet, J=7,20), 3.6 (1H, m), 3.32 (3H, s), 3.34-3.74 (6H, m), 6.88-7.10 (4H, m), 7.40 (2H, s). |
| Enantiomer | H | CH₃ | SCH₃ | Cl | (CDCl₃) δ 1.80 (3H, s), 2.48 (3H, s), 2.88 (2H, d of d, J=7.7), 3.20 (1H, m), 3.40-3.66 (5H, m), 3.79 (1H, d, J=7), 6.88-7.14 (4H, m), 7.40 (2H, s). |
| Enantiomer | CH₃ | CH₃ | SCH₃ | Cl | (CDCl₃) δ 1.96 (3H, s), 2.46 (3H, s), 2.80 (1H, ab quartet, J=7.20), 2.82 (1H, ab quartet, J=7.20) 3.16 (1H, m), 3.32 (3H, s), 3.34-3.74, (6H, m), 6.88-7.10 (4H, m), 7.40 (2H, s). |
| Racemate | H | CH₃ | SCH₃ | CF₃ | (CDCl₃) δ 2.06 (3H, s), 2.24 (3H,s), 2.70 (1H, ab quartet, J=7, 30), 2.72 (1H, ab quartet, J=7,30), 3.20 (1H, m), 3.50-3.80 (6H, m), 6.88-7.12 (4H, m), 7.65 (2H, s). |
| Racemate | CH₃ | CH₃ | SCH₃ | CF₃ | (CDCl₃) δ 2.12 (3H, s), 2.32 (3H, s), 2.78 (1H, ab quartet, J=7, 16), 2.80 (1H, ab quartet, J=7, 16), 3.18 (1H, m), 3.30 (3H, s), 3.50-3.90 (6H, m), 6.92-7.16 (4H, m), 7.64 (2H, s). |
| Racemate | H | Et | Et | Cl | (CDCl₃) δ 0.84 (3H, t, J=7), 1.22 (3H, t, J=7), 2.28 (2H, q, J=7), 2.60 (2H, q, J=7), 2.56 (1H, d of d, J=7,15), 3.26 (1H, m), 3.50-3.86 (6H, m), 6.96-7.08 (4H, m), 7.42 (2H, s). |
| Racemate | CH₃ | Et | Et | Cl | (CDCl₃) δ 0.92 (3H, t, J=7), 1.20 (3H, t, J=7), 2.38 (2H, q, J=7), 2.66 (2H, q, J=7), 2.80 (1H, ab quartet, J=7, 40), 2.82 (1H, ab quartet, J=7, 40), 3.16 (1H, m), 3.34 (3H, s), 3.35-3.74 (6H, m), 6.92-7.10 (4H, m), 7.40 (2H, s). |
| Enantiomer | H | Et | Et | Cl | (CDCl₃) δ 0.86 (3H, t, J=7), 1.20 (3H, t, J=7), 2.26 (2H, q, J=7), 2.58 (2H, q, J=7), 2.54 (1H, d of d, J=7, 15), 2.95 (1H, d of d, J= 7, 15), 3.24 (1H, m), 3.48-3.84 (6H, m), 6.90-7.08 (4H, m), 7.40 (2H, s). |

### EXAMPLE 8

The following compounds were prepared according to Examples 3 and 5.

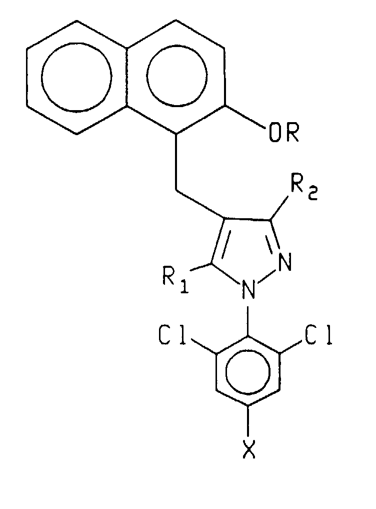

| R | R₁ | R₂ | X | ¹H-NMR |
|---|---|---|---|---|
| CH₃ | CH₃ | SCH₃ | Cl | (CDCl₃) δ 1.48 (3H, s), 2.46 (3H, s), 3.92 (3H, s), 4.14 (2H, s), 7.18-7.38 (3H, m), 7.32 (2H, s), 7.68-7.88 (3H, m). |
| CH₃ | Et | Et | CF₃ | (CDCl₃) δ 0.60 (3H, t, J=7), 1.04 (3H, t, J=7), 2.08 (2H, q, J=7), 2.46 (2H, q, J=7), 3.90 (3H, s), 4.26 (2H, s), 7.16-7.34 (3H, m), 7.58 (2H, s), 7.70-7.84 (3H, m). |
| H | CH₃ | CH₃ | Cl | (CDCl₃) δ 1.80 (3H, s), 2.10 (3H, s), 4.20 (2H, s), 6.98 (1H, d, J=7), 7.26 (1H, t, J=7), 7.36 (2H, s), 7.37 (1H, t, J=7), 7.55 (1H, d, J=7), 7.72 (1H, d, J=7), 7.78 (1H, d, J=7). |
| CH₃ | CH₃ | CH₃ | Cl | (CDCl₃) δ 1.75 (3H, s), 2.06 (3H, s), 3.94 (3H, s), 4.23 (2H, s), 7.21-7.40 (3H, m), 7.40 (2H, s), 7.71-7.86 (3H, m). |
| CH₃ | Et | Et | CF₃ | (CDCl₃) δ 0.6 (3H, t, J=7), 2.06 (3H, t, J=7), 2.08 (2H, q, J=7), 2.46 (2H, q, J=7), 3.90 (3H, s), 4.24 (2H, s), 7.18-7.36 (2H, m), 7.60 (2H, s), 7.71 (2H, d, J=8), 7.81(2H,d,J=8). |

### Example 9

### A. 3,5-Diethyl-1-(2,4,6-trimethylphenyl)pyrazole.

A solution of 7.46 g (0.04 mol) of 2,4,6-trimethylphenylhydrazine hydrochloride, 5.12 g (0.40 mol) of 3,5-heptanedione and 4.18 mL (0.60 mol) of triethylamine in 100 mL of absolute ethanol was refluxed overnight. The solvent was evaporated from the cooled reaction mixture and the residues were partitioned between water and ethyl acetate. The organic extracts were dried with brine and magnesium sulfate, and the solvent was evaporated to give the desired product in 95% yield. This compound was used in the subsequent reaction without further purification ¹H-NMR (CDCl₃): .11 (3H, t, J=7), 1.24 (3H, t, J=7), 1.90 (6H, s), 2.22 (2H, q, J-7), 2.28 (3H, s), 2.65 (2H, q, J=7), 5.96 (1H, s), 6.86 (2H, s).

### B. 4-Bromo-3,5-diethyl-1-(2,4,6-trimethylphenyl)pyrazole.

A solution of 6.4 g (0.04 mol) of bromine in 20 mL of glacial acetic acid was added dropwise to a stirred solution of 9.00 g (37 mmol) of 3,5-diethyl-1-(2,4,6-trimethylphenyl)pyrazole in 100 mL of glacial acetic acid. After 1 hour at room temperature, the acetic acid was evaporated under reduced pressure and the residues were dissolved in ethyl acetate. This solution was washed with saturated sodium bicarbonate to remove residual acetic acid, dried with brine and magnesium sulfate, and was concentrated on the rotovap. The product was a tan solid (10.26 g, purification. ¹H-NMR(CDCl₃):0.92 (3H, t, J=7), 1.15 (3H, t, J=7), 1.86 (6H, s), 2.24 (3H, s), 2.32 (2H, q, J=7), 2.60 (2H, q, J=7), 6.82 (2H, s).

### C. 3,5-Diethyl-1-(2,4,6-trimethylphenyl)pyrazole-4-methanol.

A solution of 1.0 g (3.1 mmol) of 4-bromo-3,5-diethyl-1-(2,4,6-trimethylphenyl)pyrazole in 10 mL of anhydrous ether in a flame-dried 3-neck round bottom flask under dry nitrogen with 3.85 mL of 1.7 m t-butylithium in pentane. After 1 hour, the reaction mixture was treated with 0.355 mL of ethyl chloroformate and was then allowed to warm to room temperature. The reaction mixture was quenched with water and then ethyl acetate was added. The aqueous layer was extracted with ethyl acetate again and the organic extracts were combined and dried with brine and magnesium sulfate and then the solvent was removed on the rotovap. This product, 3,5-diethyl-4-ethoxycarbonyl-1-(2,4,6-trimethylphenyl)pyrazole, was determined to be 59% pure by gas chromatographic (GC) analysis.

This material, approximately 3.1 mmol, was dissolved in 10 mL of ether and cooled under dry nitrogen to 0 °C. Then 7 mL (10 mmol) of 1.5M diisobutylaluminum hydride in tulene was added over about 10 minutes. The reaction mixture was stirred at 0 °C until no starting material was observed by GC and was then quenched with water. The product was extracted into ethyl acetate, dried with brine and magnesium sulfate, and concentrated. The residues were flash chromatographed on silica gel using 4:1 and 1:1 hexane/ethyl acetate as eluent to give the desired product as an oil in the amount of 0.565 g (69% yield for the two reactions). ¹H-NMR (CDCl₃):0.94 (3H, t, J=7), 1.23 (3H, t, J=7), 1.88 (6H, s), 2.26 (3H, s), 2.35 (2H, 1, J=7), 2.66 (2H, q, J=7), 4.50 (2H, s), 6.82 (2H, s).

### D. {2-[3,5-Diethyl-1-(2,4,6-trimethylphenyl)-1H-pyrazol-4-ylmethyl]-1,2,3,4-tetrahydroisoquinolin-3-yl}methanol.

To a solution of 272 mg (1,0 mmol) of 3,5-diethyl-1-(2,4,6-trimethylphenyl)pyrazole-4-methanol in 5 mL of methylene chloride cooled to 0°C under dry nitrogen in a 25 mL 3-neck flask, was added to 0.2 mL (2.5 mmol) of triethylamine and 0.092 mL (2.0 mmol) of methanesulfonyl chloride. This mixture was stirred for 15 minutes at 0 °C and then 0.648 g (4.0 mmol) of (+)-3-hydroxymethyl-1,2,3,4-tetrahydroisoquinoline in 1 mL of 50:50 dimethylformamide acetonitrile was added. The reaction mixture was heated at reflux overnight whereupon no starting material was seen by TLC. The cooled reaction mixture was diluted with water and the product was extracted with ethyl acetate. After drying (brine wash, magnesium sulfate) and evaporation, the crude product was chromatographed on silica gel, eluting with 10:1 and 5:1 hexane/ethyl acetate to give 184 mg (44%) of the desired product. ¹H-NMR (CDCl₃):0.80 (3H, t, J=7), 1.18 (3H, t, J=7), 1.92 (6H, s), 2.21 (2H, q, J=7), 2.28 (3H, s), 2.55 (2H, q, J=7), 2.97 (2H, d of d, J=7), 3.25 (1H, m), 3.50 - 3.66 (5H, m), 3.80 (2H, d, J=12), 6.82 - 7.16 (6H, m).

### E. 2-[3,5-Diethyl,1,(2,4,6-trimethylphenyl)-1H-pyrazol-4-ylmethyl]-3-methoxymethyl-1,2,3,4-tetrahydroisoquinoline.

A solution of 150 mg (0.36 mmol) of {2-[3,5-diethyl-1-(2,4,6-trimethylphenyl)-1H-pyrazol4-ylmethyl]-1,2,3,4-tetrahydroisoquinolin-3-yl}methanol in 5 mL of THF was stirred under dry nitrogen as 11 mg (0.43 mmol) of oil-free sodium hydride was added. The reaction mixture was stirred for 15 minutes and then 0.044 mL (0.72 mmol) of methyliodide was added. The reaction mixture was stirred overnight and then diluted with water. The product was extracted into ethyl acetate and the organic extracts were dried with brine and magnesium sulfate, and evaporated. The product was isolated pure by chromatography on silica gel using 10:1 and 5:1 hexane/ethyl acetate as eluent to give 84 mg (52%) of a golden oil. ¹H-NMR (CDCl₃):0.86 (3H, t, J=7), 1,20 (3H,t, J=7), 1.92 (6H, s), 2.28 (3H, s), 2.32 (2H, q, J=7), 2.63 (2H, q, J=7) 2.83 (2H, d of ABq), 3.17 (1H, m), 3.33 (3H, s), 3.34 - 3.38 (1H, m), 3.54 - 3.76 (5H, m), 6.83 -7.16 (6H, m).

The following examples illustrate the preparation of intermediates.

### Preparation 1

### Racemic(1,2,3,4-Tetrahydro-isoquinolin-3-yl-methanol [also referred to as (±)-3-hydroxymethyl-1,2,3,4-tetrahydroisoquinoline

To a well stirred, ice-bath-chilled slurry of 1,2,3,4-tetrahydroisoquinoline-3-carboxylic acid hydrochloride (75 g, 0.351 mol. Aldrich Chemical Co.) in anhydrous methanol (600 ml), sodium methoxide (37.92 g, 0.702 mol) was added in small solid portions over a 10 minute period. After 30 minutes of brisk stirring, the methanol was removed and the colorless residue was dried in vacuo overnight. The entire sample was stirred in anhydrous tetrahydrofuran causing the organic portion to dissolve completely. A 1.0 M solution of lithium aluminum hydride in tetrahydrofuran (351 ml, 0.351 mol) was added in a rapid stream to the well-stirred mixture over a 20 minute period (mild exotherm). The reaction mixture was then vigorously refluxed for 2 hours. At 5°C, the reaction was quenched by cautious addition of 15% aqueous sodium hydroxide. The mixture was filtered, and the filtrate was concentrated in vacuo to a yellow solid. The entire sample was then dissolved in methylene chloride (400 ml) and filtered to remove residual inorganic salts. Solvent removal in vacuo afforded the title compound as an orange solid (47.01 g, 70% yield). TLC R₁ (silica gel plates, u.v. detection, methanol/methylene chloride=5:95 in volume): 0.46; ¹³C NMR(CDCl₃): 135.4, 134.1, 129.3, 126.3, 126.1, 125.9, 65.4, 55.0, 47.8, 30.9.

### Preparation 2

### Dextrorotatory enantiomer of (1,2,3,4-Tetrahydro-isoquinolin-3-yl)-methanol (also referred to as (+)-3-hydroxymethyl-1,2,3,4-tetrahydroisoquinoline)

To a solution of (±)-3-hydroxymethyl-1 ,2,3,4-tetrahydroisoquinoline (Preparation 1; 47.01 g, 0.288 mol) in isopropyl alcohol (159 ml), a solution of (S)-(+)-mandelic acid -(43.81 g, 0.288 mol) in isopropyl alcohol (159 ml) was added. The resulting solution was allowed to stand at ambient temperature for 48 hours, during which time a heavy orange crystalline mass formed. The isolated crystalline solid (13.06 g) was dissolved in hot isopropyl alcohol (63 ml). After standing for 1 hour at ambient temperature, the newly-formed crystalline solid was isolated by filtration (8.2 g, m.p. 138°C). The recrystallization procedure was repeated twice more, using 63 ml and 60 ml volumes of isopropyl alcohol to afford 7.08 g and 6.76 g of crystalline material, respectively. (In each case, the crystallization was allowed to proceed for 2 hours at ambient temperature prior to filtration.) A 138-139°C m.p. was observed after the final crystallization. The entire sample was dissolved in methylene chloride water (300 ml and 100 ml, respectively) with the pH adjusted to 9.5 (potassium carbonate). The phases were separated, and the aqueous portion was extracted with three 50 ml portions of fresh methylene chloride. The combined organic extracts were dried (anhydrous sodium sulfate) and concentrated in vacuo to afford the optically resolved title compound as a colorless amorphous solid (2.02 g, 8.6% yield). [a]²⁰_{D} + 103° (c=1.83, CH₂Cl₂); ¹³C NMR (CDCl₃): identical to that of the racemic compound prepared in Preparation 1.

### Preparation 3

### Levorotatory enantiomer of (1,2,3,4-Tetrahydro-isoquinolin-3-yl)-methanol [also referred to as (-)-3-hydroxymethyl-1,2,3,4-tetrahydroisoquinoline]

Substituting (R)-(-)-mandelic acid for (S)-(+)-mandelic acid in the Preparation 2 procedure (and utilizing 17.9 g of the alcohol-amine prepared in Preparation 1), the levorotary title compound (0.65 g, 7.3% yield) was obtained as a colorless amorphous solid. [a]²⁰_{d}-100.4° (CH₂Cl₂, c=1.43; ¹H NMR and ¹³C NMR (CDCl₃): identical in all respects to those observed for the racemic (Preparation 1) and dextrorotatory (Preparation 2) products.

### Preparation 4

### Methyl 3,5-diethyl-1-(2,4,6-trichlorophenyl)pyrazole-4-carboxylate

A mixture of 11.0 g (60.0 mmol) of methyl 2-propionyl-3-ketopentanoate and 11.26 g (65.0 mmol) of 2,4,6-trichlorophenylhydrazine in 50 mL of ethanol was refluxed under nitrogen until disappearance of starting material was noted. The solvent was removed in vacuo and the residues were partitioned between ethyl acetate and dilute hydrogen chloride. The organic layer was dried and evaporated to give the product as an off-white solid which was used for subsequent reactions without further purification. ¹H-NMR: (CDCl₃) δ 1.02 (3H, t, J=7), 1.21 (3H, t, J=7), 2.62 (2H, q, J=7), 2.86 (2H, q, J=7), 3.82 (3H, s), 7.42 (2H, s).

## Claims

1. A compound of the formula 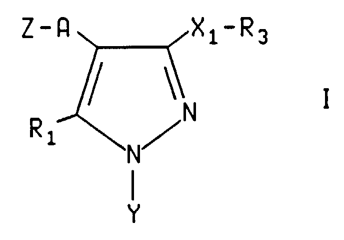 and the pharmaceutically acceptable acid addition salts thereof,
wherein
A is CH₂;
R₁ is hydrogen; linear or branched C₁-C₆ alkyl; C₃-C₆ alkyl containing one or two non-adjacent double bonds; hydroxy; O(C₁-C₆ alkyl); SH; S(C₁-C₆ alkyl); or C₃-C₆ cycloalkyl; morpholinyl, piperdinyl or aryl which aryl may be substituted by one to three of fluoro, chloro, bromo, hydroxy, O(C₁-C₆ alkyl), SH, S(C₁-C₆ alkyl), amino, NH(C₁-C₆ alkyl), N(C₁-C₆ alkyl)₂, or one of iodo, nitro or cyano, said aryl being selected from the group consisting of phenyl, thienyl, benzothienyl, pyridyl, quinolyl, pyrazinolyl, pyrimidyl, imidazolyl, benzimidazolyl, furanyl, benzofuranyl, thiazolyl, benzothiazolyl, isothiazolyl, benzoisothiazolyl, isoxazolyl, benzisoxazolyl, triazolyl, pyrazolyl, pyrrolyl, indolyl, azaindolyl, oxazolyl, benzoxazolyl, pyrrolidinyl, or thiazolidinyl;
R₃ is linear C₁-C₆ alkyl, branched C₃-C₈ alkyl, C₃-C₈ alkenyl wherein the double bond is not adjacent to X₁ when X₁ is a heteroatom, C₃-C₇ cycloalkyl(CH₂)ₙ wherein n is 0 to 4, or (CH₂)_{q}Q₁R₁₉ wherein q is 0, 1 or 2, Q₁ is O, S, NH, N(C₁-C₆ alkyl), or a covalent bond when X₁ is not a covalent bond, and R₁₉ is hydrogen, linear C₁-C₆ alkyl, branched C₃-C₈ alkyl, C₃-C₈ alkenyl, C₃-C₆ cycloalkyl or C₃-C₆ cycloalkyl- (CH₂) with the proviso that when q is 1, then X₁ and Q₁ cannot both be a heteroatom;
X₁ is a covalent bond, CH₂, O, S, or NR, wherein R is hydrogen, linear C₁-C₆ alkyl or branched C₃-C₈ alkyl;
Y is phenyl, thienyl, benzothienyl, pyridyl, quinolyl, pyrazinolyl, pyrimidyl, imidazolyl, benzimidazolyl, furanyl, benzofuranyl, thiazolyl, benzothiazolyl, isothiazolyl, benzisothiazolyl, isoxazolyl, benzisoxazolyl, triazolyl, pyrazolyl, pyrrolyl, indolyl, azaindolyl, oxazolyl, benzoxazolyl, pyrrolidinyl, thiazolidinyl, morpholinyl, or piperidinyl, each of which may be substituted by one to three of any one of fluoro, chloro, bromo, or methyl, or one of trifluoromethyl; with the proviso that Y is not unsubstituted phenyl; and
Z is 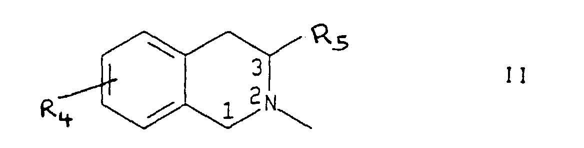
R₄ is hydrogen, C₁-C₆ alkyl, C₁-C₆ alkoxy, hydroxy, fluoro, chloro, bromo, iodo or trifluoromethyl;
R₅ is hydrogen, linear C₁-C₆ alkyl, branched C₃-C₈ alkyl, C₃-C₈ alkenyl, or (CH₂)ₒ-X₂-(CH₂)ᵣQ₂-R₆;
X₂ and Q₂ are each independently O, S, NH, N(C₁-C₆ alkyl), or one of X₂ and Q₂ may be a covalent bond;
R₆ is hydrogen, linear C₁-C₆ alkyl, branched C₃-C₈ alkyl, or C₃-C₈ alkenyl;
m is 0 or 1;
o is 1 or 2;
p is 1 or 2; and
r is 0, 1 or 2.
or z is 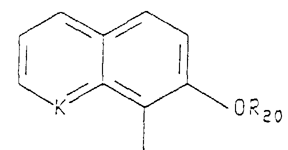
wherein K is C or N and R₂₀ is methyl, ethyl, isopropyl, cyclopropylmethylene, methoxyethylene or hydroxymethylene.

2. A compound according to claim 1 wherein Y is 2,4,6-tri-substituted phenyl.

3. A compound according to claim 1 wherein Y is 2,4,6-trichlorophenyl, 2,6-dichloro-4-trifluoromethylphenyl, 2,6-dibromo-4-fluorophenyl, 2,6-dimethyl-4-bromophenyl, or 2,4,6-trimethylphenyl.

4. A compound according to claim 1,2 or 3 wherein X₁R₃ is ethyl or methylthio.

5. A compound according to any one of claims 1 to 4 wherein R₁ is (C₁-C₆) alkyl.

6. A compound according to any one of the preceding claims wherein Z is 1,2,3,4tetrahydroisoquinolin-2-yl substituted by R₅ which is (CH₂)ₒ-X₂-(CH₂)ᵣ-Q₂-R₆.

7. A compound according to claim 6 wherein R₅ is (CH₂)ₖOH wherein k is 1 to 4, or CH₂OCH₂CH₂OR₆.

8. A compound according to any one of the preceding claims wherein Z is 1, 2, 3, 4-tetrahydroisoquinolin-2-yl, wherein R₅ is substituted at position 3, and the absolute configuration at the 3-position is S or R or R,S.

9. A compound according to any one of the preceding claims wherein Z is of the formula 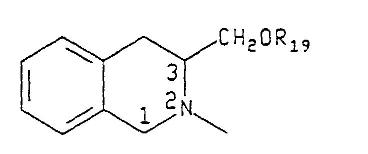 with the absolute configuration at position 3 determined by its derivation from (+)-3-hydroxymethyl-1,2,3,4-tetrahydroisoquinoline, wherein R₁₉ is methyl, ethyl, isopropyl, cyclopropylmethylene, or 2-hydroxyethyl.

10. A compound according to any one of the preceding claims wherein R₅ is CH₂OCH₃ or CH₂OCH₂CH₂OH.

11. A compound according to claim 1 which is 3-methoxymethyl-2-[5-methyl-3-methylsufanyl-1-(2,4,6-trichiorophenyl)-1H-pyrazol-4-ylmethyl]-1,2,3,4-tetrahydroisoquinoline; (3R)-3-methoxymethyl-2-[5-methyl-3-methylsulfanyl-1-(2,4,6-trichlorophenyl)-1H-pyrazol-4-ylmethyl]-1,2,3,4-tetrahydroisoquinoline; 3-methoxymethyl-2-[5-methyl-3-methylsulfanyl-1-(2,6-dichloro-4-trifluoromethylphenyl)-1H-pyrazol-4-ylmethyl]-1,2,3,4-tetrahydroisoquinoline; {2-[5-methyl-3-methylsulfanyl-1-(2,4,6-trichlorophenyl)-1H-pyrazol-4-ylmethyl]-1,2,3,4-tetrahydroisoquinolin-3-yl}methanol; {2-[5-methyl-3-methylsulfanyl-1-(2,6-dichloro-3-trifluoromethylphenyl)-1H-pyrazol-4-ylmethyl]-1,2,3,4-tetrahydroisoquinolin-3-yl)methanol; 2-[1-(2,6-dichloro-4-trifluoromethylphenyl)-3,5-diethyl-1H-pyrazol-4-ylmethyl]-naphthalene-2-yloxy)ethanol; 2-(8-[1-(2,6-dichloro-4-trifluoromethylphenyl)-3,5-diethyl-1H-pyrazol-4-ylmethyl]-quinolin-7-yloxy)-ethanol; 2-[3,5-diethyl-1-(2,4,6-trichlorophenyl)-1H-pyrazol-4-ylmethyl]-3-methoxymethyl-1,2,3,4-tetrahydroisoquinoline; 1-(2,6-dichloro-4-trifluoromethylphenyl)-3,5-diethyl-4-(2-methoxynaphthalen-1-ylmethyl)-1H-pyrazole; 2-[1-(2,6-dichloro-4-trifluoromethylphenyl)-3,5-diethyl-1H-pyrazol-4-ylmethyl]-3-methoxymethyl-1,2,3,4-tehrahydroisoquinoline, 2-[3,5-diethyl-1-(2,4,6-trimethylphenyl)-1H-pyrazol-4-ylmethyl]-3-methoxymethyl-1,2,3,4-tetrahydroisoquinoline; 2-[1-(2,6-dichloro-4-trifluoromethylphenyl)-3,5-diethyl-1H-pyrazol-4-ylmethyl]-3-ethoxymethyl-1,2,3,4-tetrahydrosioquinoline, or 2-[3,5-diethyl-1-(2,4,6-trimethylphenyl)-1H-pyrazol-4-ylmethyl]-3-ethoxymethyl-1,2,3,4-tetrahydroisoquinoline.

12. A pharmaceutical composition which comprises a compound of the formula 1 and pharmaceutical salts thereof as defined in any one of the preceding claims and a pharmaceutically acceptable carrier.

13. A compound of formula 1 as claimed in any one of the preceding claims and pharmaceutically acceptable salts thereof for use in medicine.

14. Use of a compound of formula 1 and pharmaceutical salt thereof as defined in any one of the preceding claims in the manufacture of a medicament for the treatment of (a) illnesses induced or facilitated by corticortropin releasing factor or (b) stress and anxiety related disorders including stress-induced depression and headache, adbominal bowel syndrome, inflammatory disorders, immune suppression, HIV infections, Alzheimer's disease, gastrointestinal diseases, anorexia nervosa, hemorrhagic stress, drug and alcohol withdrawal symtoms, drug addiction, and fertility problems.

15. A compound of the formula 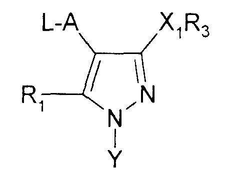 wherein
A is CH₂, R₃ is linear C₁-C₆ alkyl, branched C₃-C₈ alkyl, C₃-C₈ alkenyl wherein the double bond is not adjacent to the N or X₁ when X₁ is oxygen or sulfur, C₃-C₇ cycloalkyl (CH₂)ₙ wherein n is 0, 1, 2, 3 or 4; or (CH₂)_{q}Q₁R₆ wherein q is 0, 1 or 2, Q₁ is O, S, NH, N(C₁-C₆ alkyl) or a covalent bond, and R₆ is hydrogen, linear C₁-C₆ alkyl, branched C₃-C₈ alkyl, C₃-C₈ alkenyl, C₃-C₆ cycloalkyl, or C₃-C₆ cycloalkyl (CH₂)ₙ wherein n is 0 to 4, with the proviso that when q is 1, then X₁ and Q₁ can not both be a heteroatom;
X₁ is a covalent bond, CH₂NR, wherein R is hydrogen or linear C₁-C₆ alkyl, O, or S;
Y is phenyl, thienyl, benzothienyl, pyridyl, quinolyl, pyrazinolyl, pyrimidyl, imidazolyl, benzimidazolyl, furanyl, benzofuranyl, thiazolyl, benzothiazolyl, isothiazolyl, benzisothiazolyl, isoxazolyl, benzisoxazolyl, triazolyl, pyrazolyl, pyrrolyl, indolyl, azaindolyl, oxazolyl, benzoxazolyl, pyrrolidinyl, thiazolidnyl, morpholinyl, or piperidinyl, each of which may be substituted by one to three of any one of fluoro, chloro, bromo, or methyl, or one of trifluoromethyl, provided that Y is not unsubstituted phenyl, and
L is chloro, bromo, iodo, hydroxy, O(C=O)(C₁-C₆ alkyl), OSO₂(C₁-C₆ alkyl), OSO₂aryl wherein said aryl is phenyl which may be substituted by one to three of fluoro, chloro, bromo, hydroxy, O(C₁-C₆ alkyl), SH, S(C₁-C₆ alkyl), amino, NH(C₁-C₆ alkyl), N(C₁-C₆ alkyl)₂, or one of iodo, nitro or cyano.

## Patentansprüche

1. Verbindung der Formel 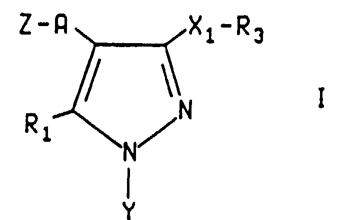 und die pharmazeutisch verträglichen Säureadditionssalze davon, worin
A CH₂ darstellt;
R₁ Wasserstoff, lineares oder verzweigtes C₁-C₆-Alkyl; C₃-C₆-Alkyl, das ein oder zwei nicht benachbarte Doppelbindungen enthält; Hydroxy; O(C₁-C₆-Alkyl); SH; S(C₁-C₆-Alkyl); oder C₃-C₆-Cycloalkyl; Morpholinyl, Piperidinyl oder Aryl, wobei dieser Arylrest mit ein bis drei Resten von Fluor, Chlor, Brom, Hydroxy, O(C₁-C₆-Alkyl), SH, S(C₁-C₆-Alkyl), Amino, NH(C₁-C₆-Alkyl), N(C₁-C₆-Alkyl)₂ oder einem der Reste Jod, Nitro oder Cyano substituiert sein kann, bedeutet, wobei der Arylrest ausgewählt ist aus der Gruppe, bestehend aus Phenyl, Thienyl, Benzothienyl, Pyridyl, Chinolyl, Pyrazinolyl, Pyrimidyl, Imidazolyl, Benzimidazolyl, Furanyl, Benzofuranyl, Thiazolyl, Benzothiazolyl, Isothiazolyl, Benzoisothiazolyl, Isoxazolyl, Benzisoxazolyl, Triazolyl, Pyrazolyl, Pyrrolyl, Indolyl, Azaindolyl, Oxazolyl, Benzoxazolyl, Pyrrolidinyl und Thiazolidinyl;
R₃ lineares C₁-C₆-Alkyl, verzweigtes C₃-C₈-Alkyl, C₃-C₈-Alkenyl, wobei die Doppelbindung zu X₁ nicht benachbart ist, wenn X₁ ein Heteroatom ist, C₃-C₇-Cycloalkyl(CH₂)ₙ, worin n 0 bis 4 ist, oder (CH₂)_{q}Q₁R₁₉ worin q 0, 1 oder 2 ist, Q₁ O, S, NH, N(C₁-C₆-Alkyl) oder eine kovalente Bindung darstellt, wenn X₁ keine kovalente Bindung darstellt, und R₁₉ Wasserstoff, lineares C₁-C₆-Alkyl, verzweigtes C₃-C₈-Alkyl, C₃-C₈-Alkenyl, C₃-C₆-Cycloalkyl oder C₃-C₆-Cycloalkyl(CH₂) darstellt, bedeutet, mit der Maßgabe, daß wenn q 1 ist, dann X₁ und Q₁ nicht beide ein Heteroatom sein dürfen;
X₁ eine kovalente Bindung, CH₂, O, S oder NR, worin R Wasserstoff, lineares C₁-C₆-Alkyl oder verzweigtes C₃-C₈-Alkyl darstellt, bedeutet;
Y Phenyl, Thienyl, Benzothienyl, Pyridyl, Chinolyl, Pyrazinolyl, Pyrimidyl, Imidazolyl, Benzimidazolyl, Furanyl, Benzofuranyl, Thiazolyl, Benzothiazolyl, Isothiazolyl, Benzisothiazolyl, Isoxazolyl, Benzisoxazolyl, Triazolyl, Pyrazolyl, Pyrrolyl, Indolyl, Azaindolyl, Oxazolyl, Benzoxazolyl, Pyrrolidinyl, Thiazolidinyl, Morpholinyl oder Piperidinyl darstellt, wobei jeder davon mit einem bis drei beliebigen Resten von Fluor, Chlor, Brom oder Methyl oder einem von Trifluormethyl substituiert sein kann, mit der Maßgabe, daß Y keinen unsubstituierten Phenylrest darstellt, und Z 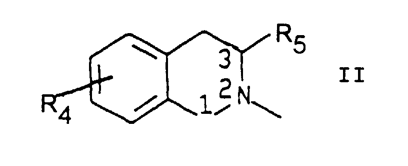 darstellt;
R₄ Wasserstoff, C₁-C₆-Alkyl, C₁-C₈-Alkoxy, Hydroxy, Fluor, Chlor, Brom, Jod oder Trifluormethyl darstellt;
R₅ Wasserstoff, lineares C₁-C₆-Alkyl, verzweigtes C₃-C₈-Alkyl, C₃-C₈-Alkenyl oder (CH₂)ₒ-X₂-(CH₂)ᵣ-Q₂-R₆ darstellt;
X₂ und Q₂ jeweils unabhängig voneinander O, S, NH, N(C₁-C₆-Alkyl) darstellen, oder einer der Reste von X₂ und Q₂ eine kovalente Bindung sein kann;
R₆ Wasserstoff, lineares C₁-C₆-Alkyl, verzweigtes C₃-C₈-Alkyl oder C₃-C₈-Alkenyl darstellt;
m 0 oder 1 ist;
o 1 oder 2 ist;
p 1 oder 2 ist und
r 0, 1 oder 2 ist,
oder Z 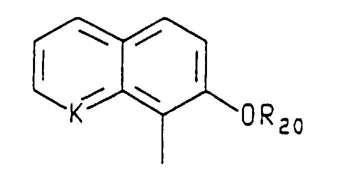 darstellt, worin K C oder N darstellt und R₂₀ Methyl, Ethyl, Isopropyl, Cyclopropylmethylen, Methoxyethylen oder Hydroxyethylen darstellt.

2. Verbindung nach Anspruch 1, worin Y 2,4,6-trisubstituiertes Phenyl darstellt.

3. Verbindung nach Anspruch 1, worin Y 2,4,6-Trichlorphenyl, 2,6-Dichlor-4-trifluormethylphenyl, 2,6-Dibrom-4-fluorphenyl, 2,6-Dimethyl-4-bromphenyl oder 2,4,6-Trimethylphenyl darstellt.

4. Verbindung nach Anspruch 1, 2 oder 3, worin X₁R₃, Ethyl oder Methylthio darstellt.

5. Verbindung nach einem der Ansprüche 1 bis 4, worin R₁ (C₁-C₆)-Alkyl darstellt.

6. Verbindung nach einem der vorangehenden Ansprüche, worin Z 1,2,3,4-Tetrahydroisochinolin-2-yl, substituiert mit R₅, das (CH₂)ₒX₂-(CH₂)ᵣ-Q₂-R₆ darstellt, ist.

7. Verbindung nach Anspruch 6, worin R₅ (CH₂)ₖOH darstellt, wobei k 1 bis 4 ist oder CH₂OCH₂CH₂OR₆ darstellt.

8. Verbindung nach einem der vorangehenden Ansprüche, worin Z 1,2,3,4-Tetrahydroisochinolin-2-yl darstellt, wobei R₅ in Stellung 3 substituiert ist und die absolute Konfiguration in der 3-Stellung S oder R oder R,S ist.

9. Verbindung nach einem der vorangehenden Ansprüche, worin Z die Formel 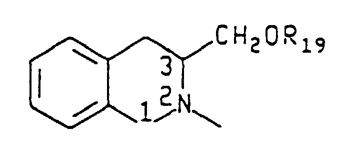 aufweist mit der absoluten Konfiguration in Stellung 3, bestimmt durch ihre Derivatisierung von (+)-3-Hydroxymethyl-1,2,3,4-tetrahydroisochinolin, wobei R₁₉ Methyl, Ethyl, Isopropyl, Cyclopropylmethylen oder 2-Hydroxyethyl darstellt.

10. Verbindung nach einem der vorangehenden Ansprüche, worin R₅ CH₂OCH₃ oder CH₂OCH₂CH₂OH darstellt.

11. Verbindung nach Anspruch 1, nämlich 3-Methoxymethyl-2-[5-methyl-3-methylsulfanyl-1-(2,4,6-trichlorphenyl)-1H-pyrazol-4-ylmethyl]-1,2,3,4-tetrahydroisochinolin, (3R)-3-Methoxymethyl-2-[5-methyl-3-methylsulfanyl-1-(2,4,6-trichlorphenyl)-1H-pyrazol-4-ylmethyl]-1,2,3,4-tetrahydroisochinolin, 3-Methoxymethyl-2-[5-methyl-3-methylsulfanyl-1-(2,6-dichlor-4-trifluormethylphenyl)-1H-pyrazol-4-ylmethyl]-1,2,3,4-tetrahydroisochinolin, {2-[5-Methyl-3-methylsulfanyl-1-(2,4,6-trichlorphenyl)-1H-pyrazol-4-ylmethyl]-1,2,3,4-tetrahydroisochinolin-3-yl}methanol, {2-[5-Methyl-3-methylsulfanyl-1-(2,6-dichlor-4-trifluormethylphenyl)-1H-pyrazol-4-ylmethyl]-1,2,3,4-tetrahydroisochinolin-3-yl}methanol, 2-[1-(2,6-Dichlor-4-trifluormethylphenyl)-3,5-diethyl-1H-pyrazol-4-ylmethyl]naphthalin-2-yloxy)ethanol, 2(8-[1-(2,6-Dichlor-4-trifluormethylphenyl)-3,5-diethyl-1H-pyrazol-4-ylmethyl]chinolin-7-yloxy)ethanol, 2-[3,5-Diethyl-1-(2,4,6-trichlorphenyl)-1H-pyrazol-4-ylmethyl]-3-methoxymethyl-1,2,3,4-tetrahydroisochinolin, 1-(2,6-Dichlor-4-trifluormethylphenyl)-3,5-diethyl-4-(2-methoxynaphthalin-1-ylmethyl)-1H-pyrazol, 2-[1-(2,6-Dichlor-4-trifluormethylphenyl)-3,5-diethyl-1H-pyrazol-4-ylmethyl]-3-methoxymethyl-1,2,3,4-tetrahydroisochinolin, 2-[3,5-Diethyl-1-(2,4,6-trimethylphenyl)-1H-pyrazol-4-ylmethyl]-3-methoxymethyl-1,2,3,4-tetrahydroisochinolin, 2-[1-(2,6-Dichlor-4-trifluormethylphenyl)-3,5-diethyl-1H-pyrazol-4-ylmethyl]-3-ethoxymethyl-1,2,3,4-tetrahydroisochinolin oder 2-[3,5-Diethyl-1-(2,4,6-trimethylphenyl)-1H-pyrazol-4-ylmethyl]-3-ethoxymethyl-1,2,3,4-tetrahydroisochinolin.

12. Pharmazeutische Zusammensetzung, die eine Verbindung der Formel I und pharmazeutische Salze davon, nach einem der vorangehenden Ansprüche und einen pharmazeutisch verträglichen Träger umfaßt.

13. Verbindung der Formel I nach einem der vorangehenden Ansprüche und pharmazeutisch verträgliche Salze davon zur Verwendung in der Medizin.

14. Verwendung einer Verbindung der Formel I und ein pharmazeutisches Salz davon nach einem der vorangehenden Ansprüche bei der Herstellung eines Arzneimittels zur Behandlung von (a) Erkrankungen, die durch Corticotropin-Freisetzungsfaktor induziert oder erleichtert werden, oder (b) durch Belastung und Angst bedingte Erkrankungen einschließlich durch Belastung induzierte Depression und Kopfschmerz, abdominales Darmsyndrom, entzündliche Erkrankungen, Immunsuppression, HIV-Infektionen, Alzheimer Krankheit, gastrointestinale Krankheiten, Anorexia nervosa, hämorrhagische Belastung, Arzneimittel- und Alkoholentzugssymptome, Drogensucht und Fertilitätsprobleme.

15. Verbindung der Formel 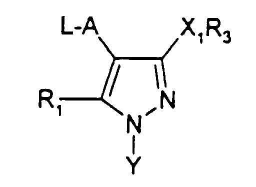 worin A CH₂ darstellt, R₃ lineares C₁-C₆-Alkyl, verzweigtes C₃-C₈-Alkyl, C₃-C₈-Alkenyl, wobei die Doppelbindung nicht zu dem N oder X₁ benachbart ist, wenn X₁ Sauerstoff oder Schwefel darstellt, C₃-C₇-Cycloalkyl(CH₂)ₙ, worin n 0, 1, 2, 3 oder 4 ist oder (CH₂)_{q}Q₁R₆ bedeutet, worin q 0, 1 oder 2 ist, Q₁ O, S, NH, N(C₁-C₆-Alkyl) oder eine kovalente Bindung darstellt, und R₆ Wasserstoff, lineares C₁-C₆-Alkyl, verzweigtes C₃-C₈-Alkyl, C₃-C₈-Alkenyl, C₃-C₆-Cycloalkyl oder C₃-C₆-Cycloalkyl(CH₂)ₙ, worin n 0 bis 4 ist, darstellt, mit der Maßgabe, daß wenn q 1 ist, X₁ und Q₁ dann nicht beide ein Heteroatom sein können;
X₁ eine kovalente Bindung, CH₂NR, worin R Wasserstoff oder lineares C₁-C₆-Alkyl bedeutet, O oder S darstellt;
Y Phenyl, Thienyl, Benzothienyl, Pyridyl, Chinolyl, Pyrazinolyl, Pyrimidyl, Imidazolyl, Benzimidazolyl, Furanyl, Benzofuranyl, Thiazolyl, Benzothiazolyl, Isothiazolyl, Benzisothiazolyl, Isoxazolyl, Benzisoxazolyl, Triazolyl, Pyrazolyl, Pyrrolyl, Indolyl, Azaindolyl, Oxazolyl, Benzoxazolyl, Pyrrolidinyl, Thiazolidinyl, Morpholinyl oder Piperidinyl, wobei jeder davon mit einem bis drei beliebigen Resten von Fluor, Chlor, Brom oder Methyl oder einem Rest von Trifluormethyl substituiert sein kann, darstellt, mit der Maßgabe, daß Y keinen unsubstituierten Phenylrest darstellt und
L Chlor, Brom, Jod, Hydroxy, O(C=O)(C₁-C₆-Alkyl), OSO₂(C₁-C₆-Alkyl), OSO₂Aryl, worin das Aryl Phenyl darstellt, das mit einem bis drei Resten von Fluor, Chlor, Brom, Hydroxy, O(C₁-C₆-Alkyl), SH, S(C₁-C₆-Alkyl), Amino, NH(C₁-C₆-Alkyl), N(C₁-C₆-Alkyl)₂ oder einem der Reste von Jod, Nitro oder Cyano substituiert sein kann, darstellt.

## Revendications

1. Composé de formule 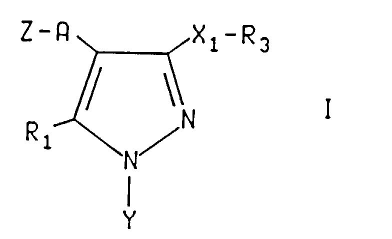 et ses sels d'addition d'acide pharmaceutiquement acceptables,
formule dans laquelle
A représente un groupe CH₂ ;
R₁ représente l'hydrogène ; un groupe alkyle en C₁ à C₆ linéaire ou ramifié ; alkyle en C₃ à C₆ contenant une ou deux doubles liaisons non adjacentes ; hydroxy ; O(alkyle en C₁ à C₆) ; SH ; S(alkyle en C₁ à C₆) ou cycloalkyle en C₃ à C₆ ; morpholinyle, pipéridinyle ou aryle, ledit groupe aryle pouvant être substitué avec un à trois substituants fluoro, chloro, bromo, hydroxy, O(alkyle en C₁ à C₆, SH, S(alkyle en C₁ à C₆), amino, NH(alkyle en C₁ à C₆) ou N(alkyle en C₁ à C₆)₂ ou un substituant iodo, nitro ou cyano, ledit groupe aryle étant choisi dans le groupe consistant en les groupes phényle, thiényle, benzothiényle, pyridyle, quinolyle, pyrazinolyle, pyrimidyle, imidazolyle, benzimidazolyle, furanyle, benzofuranyle, thiazolyle, benzothiazolyle, isothiazolyle, benzoisothiazolyle, isoxazolyle, benzisoxazolyle, triazolyle, pyrazolyle, pyrrolyle, indolyle, azaindolyle, oxazolyle, benzoxazolyle, pyrrolidinyle et thiazolidinyle ;
R₃ représente un groupe alkyle en C₁ à C₆ linéaire, alkyle en C₃ à C₆ ramifié, alcényle en C₃ à C₆ dans lequel la double liaison n'est pas adjacente à X₁ lorsque X₁ représente un hétéro-atome, (cycloalkyle en C₃ à C₇)-(CH₂)ₙ dans lequel n a une valeur de 0 à 4, ou (CH₂)_{q}Q₁R₁₉ dans lequel q est égal à 0, 1 ou 2, Q₁ représente O, S, un groupe NH, N(alkyle en C₁ à C₆) ou une liaison covalente lorsque X₁ ne représente pas une liaison covalente, et R₁₉ représente l'hydrogène, un groupe alkyle en C₁ à C₆ linéaire, alkyle en C₃ à C₈ ramifié, alcényle en C₃ à C₈, cycloalkyle en C₃ à C₆ ou (cycloalkyle en C₃ à C₆)-(CH₂), sous réserve que, lorsque q est égal à 1, alors X₁ et Q₁ ne puissent représenter l'un et l'autre un hétéro-atome ;
X₁ représente une liaison covalente, un groupe CH₂, O, S ou NR, dans lequel R représente l'hydrogène, un groupe alkyle en C₁ à C₆ linéaire ou alkyle en C₃ à C₈ ramifié ;
Y représente un groupe phényle, thiényle, benzothiényle, pyridyle, quinolyle, pyrazinolyle, pyrimidyle, imidazolyle, benzimidazolyle, furanyle, benzofuranyle, thiazolyle, benzothiazolyle, isothiazolyle, benzisathiazolyle, isoxazolyle, benzisoxazolyle, triazolyle, pyrazolyle, pyrrolyle, indolyle, azaindolyle, oxazolyle, benzoxazolyle, pyrrolidinyle, thiazolidinyle, morpholinyle, ou pipéridinyle, chacun de ces groupes pouvant être substitué avec un à trois quelconques des groupes fluoro, chloro, bromo et méthyle, ou avec un groupe trifluorométhyle ; sous réserve que Y ne représente pas un groupe phényle non substitué ; et
Z représente un groupe 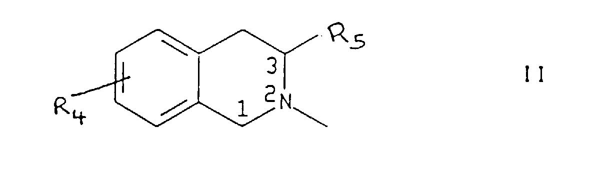
R₄ représente l'hydrogène, un groupe alkyle en C₁ à C₆, alkoxy en C₁ à C₆, hydroxy, fluoro, chloro, bromo, iodo ou trifluorométhyle ;
R₅ représente l'hydrogène, un groupe alkyle en C₁ à C₆ linéaire, alkyle en C₃ à C₈ ramifié, alcényle en C₃ à C₈ ou (CH₂)ₒ-X₂-(CH₂)ᵣ-Q₂-R₆ ;
X₂ et Q₂ représentent chacun indépendamment O, S, un groupe NH ou N(alkyle en C₁ à C₆), ou bien un des groupes de X₂ et Q₂ peut représenter une liaison covalente
R₆ représente l'hydrogène, un groupe alkyle en C₁ à C₆ linéaire, alkyle en C₃ à C₈ ramifié ou alcényle en C₃ à C₈ ;
m est égal à 0 ou 1 ;
o est égal à 1 ou 2 ;
p est égal à 1 ou 2 ;
r est égal à 0, 1 ou 2 ;
ou bien z représente un groupe 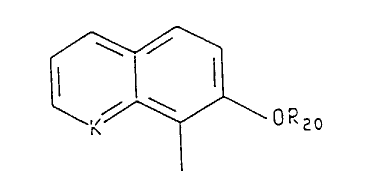
dans lequel K représente C ou N et R₂₀ représente un groupe méthyle, éthyle, isopropyle, cyclopropylméthylène, méthoxyéthylène ou hydroxyéthylène.

2. Composé suivant la revendication 1, dans laquelle Y représente un groupe phényle 2,4,6-trisubstitué.

3. Composé suivant la revendication 1, dans laquelle Y représente un groupe 2,4,6-trichlorophényle, 2,6-dichloro-4-trifluorométhylphényle, 2,6-dibromo-4-fluorophényle, 2,6-diméthyl-4-bromophényle ou 2,4,6-triméthylphényle.

4. Composé suivant la revendication 1, 2 ou 3, dans laquelle X₁R₃ représente un groupe éthyle ou méthylthio.

5. Composé suivant l'une quelconque des revendications 1 à 4, dans lequel R₁ représente un groupe alkyle en C₁ à C₆.

6. Composé suivant l'une quelconque des revendications précédentes, dans lequel Z représente un groupe 1,2,3,4-tétrahydroisoquinoléine-2-yle substitué avec un groupe R₅ qui consiste en un groupe (CH₂)o-X₂-(CH₂)ᵣ-Q₂-R₆.

7. Composé suivant la revendication 6, dans lequel R₅ représente un groupe (CH₂)ₖOH dans lequel k a une valeur de 1 à 4 ou un groupe CH₂OCH₂CH₂OR₆.

8. Composé suivant l'une quelconque des revendications précédentes, dans lequel Z représente un groupe 1,2,3,4-tétrahydroisoquinoléine-2-yle, R₅ est substitué en position 3 et la configuration absolue en position 3 et la configuration S ou R ou bien R,S.

9. Composé suivant l'une quelconque des revendications précédentes, dans lequel Z répond à la formule 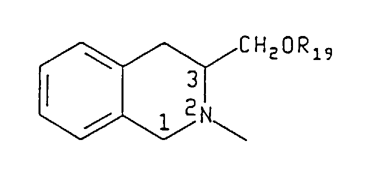 avec la configuration absolue en position 3 déterminée par la (+)-3-hydroxyméthyl-1,2,3,4-tétrahydroisoquinoléine de laquelle ils dérivent, R₁₉ représente un groupe méthyle, éthyle, isopropyle, cyclopropylméthylène ou 2-hydroxyéthyle.

10. Composé suivant l'une quelconque des revendications précédentes, dans lequel R₅ représente un groupe CH₂OCH₃ ou CH₂OCH₂CH₂OH.

11. Composé suivant la revendication 1, qui consiste en : 3-méthoxyméthyl-2-[5-méthyl-3-méthylsulfanyl-1-(2,4,6-trichlorophényl)-1H-pyrazole-4-yl-méthyl]-1,2,3,4-tétrahydroisoquinoléine ; (3R)-3-méthoxy-méthyl-2-[5-méthyl-3-méthylsulfanyl-1-(2,4,6-trichloro-phényl)-1H-pyrazole-4-ylméthyl]-1,2,3,4-tétrahydroisoquinoléine ; 3-méthoxyméthyl-2-[5-méthyl-3-méthylsulfanyl-1-(2,6-dichloro-4-trifluorométhylphényl)-1H-pyrazole-4-yl-méthyl]-1,2,3,4-tétrahydroisoquinoléine ; {2-[5-méthyl-3-méthylsulfanyl-1-(2,4,6-trichlorophényl)-1H-pyrazole-4-ylméthyl]-1,2,3,4-tétrahydroisoquinoléine-3-yl)méthanol ; {2-[5-méthyl-3-méthylsulfanyl-1-(2,6-dichloro-4-trifluorométhylphényl)-1H-pyrazole-4-ylméthyl]-1,2,3,4-tétrahydroisoquinoléine-3-yl}-méthanol ; 2-[1-(2,6-dichloro-4-trifluorométhylphényl)-3,5-diéthyl-1H-pyrazole-4-ylméthyl]-naphtalène-2-yloxyéthanol ; 2-(8-[1-(2,6-dichloro-4-trifluorométhylphényl)-3,5-diéthyl-1H-pyrazole-4-ylméthyl]-quinoléine-7-yloxy)éthanol ; 2-[3,5-diéthyl-1-(2,4,6-trichlorophényl)-1H-pyrazole-4-ylméthyl]-3-méthoxy-méthyl-1,2,3,4-tétrahydroisoquinoléine ; 1-(2,6-dichloro-4-trifluorométhylphényl)-3,5-diéthyl-4-(2-méthoxynaphtalène-1-ylméthyl)-1H-pyrazole; 2-[1-(2,6-dichloro-4-trifluoro-méthylphényl)-3,5-diéthyl-1H-pyrazole-4-ylméthyl]-3-méthoxyméthyl-1,2,3,4-tétrahydroisoquinoléine ; 2-[3,5-diéthyl-1-(2,4,6-triméthyl-phényl)-1H-pyrazole-4-ylméthyl]-3-méthoxyméthyl- 1,2,3,4-tétrahydroisoquinoléine ; 2-[1-(2,6-dichloro-4-trifluorométhylphényl)-3,5-diéthyl-1H-pyrazole-4-ylméthyl]-3-éthoxyméthyl-1,2,3,4-tétrahydroisoquinoléine ; ou 2-[3,5-diéthyl-1-(2,4,6-triméthylphényl)-1H-pyrazole-4-ylméthyl]-3'-éthoxyphméthyl-1,2,3,4-tétrahydroisoquinoléine.

12. Composition pharmaceutique, qui comprend un composé de formule I ou un de ses sels pharmaceutiquement acceptables répondant à la définition suivant l'une quelconque des revendications précédentes et un support pharmaceutiquement acceptable.

13. Composé de formule I suivant l'une quelconque des revendications précédentes et ses sels pharmaceutiquement acceptables, pour une utilisation en médecine.

14. Utilisation d'un composé de formule I ou d'un de ses sels pharmaceutiques répondant à la définition suivant l'une quelconque des revendications précédentes dans la production d'un médicament au traitement (a) de maladies induites ou facilitées par le facteur de libération de corticotrophine ou (b) de troubles liés au stress et à l'anxiété comprenant la dépression induite par le stress et les céphalées, le syndrome abdominal du colon irritable, des troubles inflammatoires, une immunosuppression, des infections par le HIV, la maladie d'Alzheimer, des maladies gastro-intestinales, l'anorexie mentale, le stress hémorragique, les symptômes de sevrage de médicaments et de l'alcool, l'accoutumance à des médicaments et des problèmes de fécondité.

15. Composé de formule 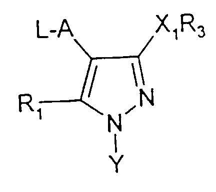 dans laquelle A représente un groupe CH₂, R₃ représente un groupe alkyle en C₁ à C₆ linéaire, alkyle en C₃ à C₈ ramifié, alcényle en C₃ à C₈ dans lequel la double liaison n'est pas adjacente à l'atome N ou X₁ lorsque X₁ représente l'oxygène ou le soufre, (cycloalkyle en C₃ à C₇)-(CH₂)ₙ dans lequel n est égal à 0, 1, 2, 3 ou 4 ; ou (CH₂)_{q}Q₁R₆ dans lequel q est égal à 0, 1 ou 2, Q₁ représente O, S, un groupe NH, N(alkyle en C₁ à C₆) ou une liaison covalente, et R₆ représente l'hydrogène, un groupe alkyle en C₁ à C₆ linéaire, alkyle en C₃ à C₈ ramifié, alcényle en C₃ à C₈, cycloalkyle en C₃ à C₆ ou (cycloalkyle en C₃ à C₆)-(CH₂)ₙ dans lequel n a une valeur de 0 à 4, sous réserve que, lorsque q est égal à 1, alors X₁ et Q₁ ne puissent représenter l'un et l'autre un hétéro-atome ;
X₁ représente une liaison covalente, un groupe CH₂NR, dans lequel R représente l'hydrogène ou un groupe alkyle en C₁ à C₆ linéaire, O ou S ;
Y représente un groupe phényle, thiényle, benzothiényle, pyridyle, quinolyle, pyrazinolyle, pyrimidyle, imidazolyle, benzimidazolyle, furanyle, benzofuranyle, thiazolyle, benzothiazolyle, isothiazolyle, benzisathiazolyle, isoxazolyle, benzisoxazolyle, triazolyle, pyrazolyle, pyrrolyle, indolyle, azaindolyle, oxazolyle, benzoxazolyle, pyrrolidinyle, thiazolidinyle, morpholinyle ou pipéridinyle, chacun de ces groupes pouvant être substitué avec un à trois quelconques des substituants fluoro, chloro, bromo et méthyle ou un substituant trifluorométhyle, sous réserve que Y ne représente pas un groupe phényle non substitué, et
L représente un groupe chloro, bromo, iodo, hydroxy, O(C=O)-(alkyle en C₁ à C₆), OSO₂(alkyle en C₁ à C₆), OSO₂aryle dans lequel ledit groupe aryle est un groupe phényle qui peut être substitué avec un à trois substituants fluro, chloro, bromo, hydroxy, O(alkyle en C₁ à C₆), SH, S(alkyle en C₁ à C₆), amino, NH(alkyle en C₁ à C₆), N(alkyle en C₁ à C₆)₂, ou avec un substituant iodo, nitro ou cyano.
